# EUROPEAN PATENT APPLICATION

(11) **EP 2 821 027 A2**
(43) Date of publication of application: **07.01.2015**
(21) Application number: 14187012.1
(22) Date of filing: 25.01.2008
(51) Int. Cl.: A61C 5/02, A61C 19/04

(54) **Apparatus for removing organic material from a root canal of a tooth**

(30) Priority: 25.01.2007 US 897343 P; 29.05.2007 US 940682 P
(62) Divisional of application: 08728345.3
(71) Applicant: SONENDO, INC., Laguna Hills, CA 92653 (US)
(72) Inventor: Gharib, Morteza, San Marino, CA California 91108 (US); Adams, Joshua, Dana Point, CA California 92629 (US); Bergheim, Bjarne, Mission Viejo, CA California 92692 (US); Piotrowski, Adam, E, Irvine, CA California 92614 (US)
(74) Representative: Carpintero Lopez, Francisco

(57) **Abstract**

An apparatus for removing organic material from a root canal of a tooth includes a liquid jet assembly, a sensor and a controller. The liquid jet assembly is configured to produce a high velocity beam of liquid capable of cleaning the root canal of organic material. The sensor is configured to detect completion of the cleaning and to produce a signal in response. The controller is configured to communicate a shut-off signal to the liquid jet assembly upon receipt of the signal from the sensor based on an acoustic signature. The acoustic signature comprises cavitation induced effects how an amplitude of the signal changes during cleaning, a high frequency acoustic signal or a low frequency acoustic signal.

## Description

### CROSS-REFERENCE TO RELATED APPLICATIONS

This application claims the benefit under 35 U.S.C. § 119(e) of U.S. Provisional Patent Application No. 60/897,343, filed January 25, 2007, entitled "METHOD AND APPARATUS FOR MONITORING CERTAIN DENTAL DRILLING PROCEDURES" and U.S. Provisional Patent Application No. 60/940,682, filed May 29, 2007, entitled "APPARATUS AND METHODS FOR ACOUSTIC SENSING OF A TOOTH." The entire disclosure of each of the above-referenced provisional patent applications is hereby incorporated by reference herein.

### BACKGROUND

### Field

The present disclosure relates to apparatus and methods for monitoring a tooth and, in some embodiments, for sensing acoustic energy propagating from a tooth and/or from regions near a tooth.

### Description of the Related Art

In conventional root canal procedures, an opening is drilled through the crown of a diseased tooth, and endodontic files are inserted into the root canal system to open the canal spaces and remove organic material therein. The root canal is then filled with solid matter such as gutta percha, and the tooth is restored. However, this procedure will not remove all organic material from the canal spaces, which can lead to post-procedure complications such as infection. In addition, motion of the endodontic file may force organic material through an apical opening into periapical tissues. In some cases, an end of the endodontic file itself may pass through the apical opening. Such events may result in trauma to the soft tissue near the apical opening and lead to post-procedure complications.

### SUMMARY

Various non-limiting aspects of the present disclosure will now be provided to illustrate features of the disclosed apparatus and methods. For example, in one aspect of the present disclosure, a detector may be configured to detect the presence of acoustic energy at an apical opening of a tooth. The acoustic energy may be produced by the impact of a liquid jet against the tooth. In one embodiment, the detector comprises a hydrophone. Preferably, the detector has a resolution, sensitivity, and detection threshold capable of detecting the acoustic energy prior to liquid or other material emerging from the apical opening due to action of the jet.

Another aspect of the present disclosure comprises a motion detector which detects motion at an apex of a tooth in situ. Preferably, the motion detector comprises an ultrasound transmitter/receiver. The preferred embodiment includes means for automatically generating a shut off signal for a liquid jet device configured to produce a liquid beam that cleans soft tissue and/or necrotic material from root canal spaces. In some embodiments, the motion detector comprises an acoustic detector configured to provide a signal in response to detection of acoustic energy from the tooth and a processor configured to receive the signal and, based at least in part on the signal, to detect motion of material near the apex of the tooth. The processor may be configured to generate a shut off signal for the liquid jet device if the motion is detected. In some embodiments, the acoustic detector comprises a bimodal acoustic sensor capable of detecting a low frequency acoustic range below about 20 kHz and a high frequency acoustic range above about 20 kHz. In some embodiments, the high frequency acoustic range includes frequencies from about 200 kHz to about 25 MHz.

Another aspect of the present disclosure comprises a method for detecting motion at an apex of a tooth. Preferably, the detection occurs during cleaning of root canals using a liquid jet. In a preferred method, the liquid jet is automatically shut off if motion is detected at an apex of the tooth (such motion being indicative of liquid passing out of an apical opening). In one embodiment, apices of a tooth are imaged, for example by ultrasound, and Doppler shifts are detected. Software may be used to specify a detection target area smaller than the imaged area in order to limit detection of motion to the area immediately surrounding such apical openings.

In another aspect of the present disclosure, an apparatus for removing organic material from a tooth comprises an energy generator configured to create cavitation within the tooth. The cavitation may produce acoustic signals. The apparatus also comprises an acoustic receiver configured to detect cavitation-induced acoustic signals that propagate from the tooth to the receiver during coupling of the energy to the tooth. In some embodiments, the cavitation-induced acoustic signals comprised frequencies in the range from about 200 kHz to about 25 MHz.

In another aspect, an apparatus for removing organic material from a tooth comprises an acoustic energy generator configured to couple acoustic energy to a dentinal surface of a tooth. The acoustic energy may be sufficient to cause organic material in the tooth to be detached from surrounding dentin. The apparatus also comprises an acoustic receiver configured to detect acoustic energy that propagates from the tooth during coupling of the acoustic energy to the tooth. In certain embodiments, the acoustic energy is sufficient to cause organic material to be detached from surrounding dentin from locations remote from the acoustic coupling surface. In other embodiments, the acoustic energy comprises energy with frequencies in a range from about 10 Hz to about 10 kHz. The frequency range may be from about 500 Hz to 5 kHz. In other embodiments, the acoustic energy comprises frequencies in a range from about 200 kHz to about 25 MHz. In certain embodiments, the frequency range extends to about 1 GHz. In certain embodiments, the acoustic energy may be produced by cavitation-induced effects including cavitation bubbles and cavitation jets.

In another aspect, an apparatus for removing organic material from a tooth is provided. The apparatus comprises a first acoustic energy generator configured to couple first acoustic energy to a dentinal surface of a tooth. The first acoustic energy may be sufficient to cause organic material in the tooth to be detached from surrounding dentin. The apparatus further comprises a second acoustic energy generator configured to couple second acoustic energy into the tooth for propagation therein. The apparatus also comprises an acoustic receiver configured to detect at least a portion of the second acoustic energy that propagates from the tooth. In certain embodiments, the first acoustic energy is sufficient to cause organic material to be detached from surrounding dentin at locations remote from the acoustic coupling surface. In some embodiments, the acoustic receiver is configured to detect second acoustic energy during coupling of the first acoustic energy to the tooth. In some embodiments, the second acoustic energy comprises frequencies in a range from about 250 kHz to about 25 MHz. In certain embodiments, the second acoustic generator and the acoustic receiver are the same structure. The structure may include an ultrasonic transducer. The first acoustic generator and the second acoustic generator may be different structures in some embodiments. In some embodiments, the second acoustic energy comprises information related to structural integrity of the tooth and/or information related to dentinal thickness. In some embodiments, the information comprises an acoustic propagation time difference between the first dentinal surface and a second dentinal surface of the tooth.

In another aspect of the present disclosure, a method comprises detaching organic material within a root canal from surrounding dentin. The method also comprises detecting a detachment event by detecting acoustic signals propagating from the tooth. The detachment event may be defined by a change in an energy responsive characteristic of such detachment. In some embodiments, the method further comprises producing a control signal in response to the detection of the detachment event and, in some embodiment, in response to the control signal, shutting off an energy source responsible for providing energy for detaching the organic material within the root canal. The energy responsive characteristic may be associated with detected acoustic energy.

In another aspect of the present disclosure, a method comprises cleaning a root canal of a tooth by applying sufficient energy to detach organic material within the root canal from surrounding dentin. The method further comprises monitoring an energy responsive characteristic associated with the cleaning during application of the energy so as to detect a detachment event defined by a change in the energy responsive characteristic. The method further comprises automatically producing a control signal in response to the detection of the detachment event to terminate application of the detachment energy. In some embodiments, the energy responsive characteristic comprises an acoustic signature of an acoustic signal propagating from the tooth. The acoustic signature may be associated with a frequency spectrum of the acoustic signal. The frequency spectrum may comprise frequencies in a range from about 10 Hz to about 10 kHz, from about 200 kHz to about 25 MHz, or some other suitable frequency range. In some embodiments, applying sufficient energy to detach organic material within the root canal from surrounding dentin comprises directing a high-velocity liquid jet to a surface of the tooth. In some embodiments, before cleaning the root canal system, the method further comprises impacting the tooth with a low-velocity liquid jet, detecting acoustic energy propagating from the tooth in response to impact of the low-velocity liquid jet, and actuating the high-velocity liquid jet in response to detecting the acoustic energy.

In another aspect, an apparatus for removing organic material from a root canal is disclosed. The apparatus comprises a liquid jet assembly configured to produce a high velocity beam of liquid capable of cleaning the root canal of organic material, a sensor configured to detect completion of the cleaning and, in response, to produce a signal. The apparatus also includes a controller configured to automatically terminate the high velocity beam upon receipt of the signal from the sensor. In some embodiments, the sensor comprises an acoustic sensor.

In another aspect, an acoustic detector that is capable of being acoustically coupled to a tooth is responsive to a low frequency acoustic range below about 20 kHz and a high frequency acoustic range above about 20 kHz. The high frequency acoustic range may comprise frequencies from about 200 kHz to about 25 MHz.

In another aspect, a method for acoustically coupling an acoustic element to a tooth is provided. The method comprises positioning an end of an acoustic element near a surface of a tooth, disposing a flowable material between the end of the acoustic element and the surface of the tooth, and hardening the flowable material. In some embodiments, the hardened material acts as an acoustic waveguide for acoustic energy propagating between the tooth and the acoustic element. In some embodiments, the acoustic element comprises a housing, and disposing comprises disposing the flowable material in the housing. In some embodiments, hardening comprises light curing the flowable material. In some embodiments, the flowable material comprises a flowable composite comprising a filler material. In some embodiments, the method further comprises selecting a fractional amount of the filler material in the composite to provide a desired acoustic impedance of the hardened material.

In another aspect, a dental instrument comprises a first nozzle configured to output a first liquid beam and a second nozzle configured to output a second liquid beam that intersects the first liquid beam at a distance from the first nozzle. In some embodiments, the first liquid beam comprises a high-velocity liquid jet. In some embodiments, the second liquid beam comprises a low-velocity liquid jet. In some embodiments, the distance is adjustable. In some embodiments, the distance is in a range from about 5 mm to about 50 mm. In some embodiments, the first liquid beam and the second liquid beam intersect at an angle. The angle may be in a range from about 1 degree to about 10 degrees.

In another aspect, a dental instrument comprises a nozzle configured to output a liquid beam and an aiming element having an end portion configured to contact a region of a tooth such that when the end portion contacts the region of the tooth, the nozzle is a predetermined distance from the region. In some embodiments, the liquid beam comprises a high-velocity, collimated liquid jet. In some embodiments, the aiming element comprises an elongated member. In some embodiments, the elongated member is offset from a propagation axis of the liquid beam. In some embodiments, the elongated member comprises a portion having a lumen, and the liquid beam is configured to pass through the lumen. In some embodiments, the end portion has a rounded tip, an elongated tip, and/or a frustoconical tip. In some embodiments, the predetermined distance is in a range from about 5 mm to about 50 mm.

In another aspect, an aiming element for use with a handpiece having a nozzle capable of outputting a liquid jet is provided. The aiming element comprises an elongated member having a distal end capable of contacting a location on a tooth and a proximal end capable of attachment to the handpiece. The aiming element is configured such that when it is attached to the handpiece, the elongated member does not impede propagation of the liquid jet, and when the distal end contacts the location on the tooth, the nozzle is a predetermined distance from the location. In some embodiments, the elongated member comprises a portion having a lumen, and the liquid jet is capable of passing through the lumen. In some embodiments, the distal end comprises a rounded tip, an elongated tip, and/or a frustoconical tip. In some embodiments, the predetermined distance is in a range from about 5 mm to about 50 mm.

In another aspect, a method for monitoring a tooth in a patient's mouth comprises directing a low-velocity liquid jet toward a location in a tooth, detecting whether liquid from the liquid jet is present at the location of the tooth, generating a signal in response to the detection, and actuating a high-velocity liquid jet in response to the generated signal. In some embodiments, the low-velocity jet has insufficient energy to cut tissue in the patient's mouth. In some embodiments, detecting whether liquid from the liquid jet is present comprises detecting acoustic energy caused by impact of the low-velocity jet. In some embodiments, detecting whether liquid from the liquid jet is present comprises detecting motion of liquid from the low-velocity liquid jet. In some embodiments, the high-velocity liquid jet has sufficient energy to cut tissue in the patient's mouth.

In another aspect, a strain gage for monitoring a tooth comprises a member configured to be at least partially inserted into an opening in the tooth, and a strain-sensing element coupled to the member. The strain-sensing element is configured to generate a signal in response to deformation of the strain-sensing element caused by movement of the member. In some embodiments, the member comprises an elongated element having a proximal end and a distal end. The proximal end is coupled to the strain-sensing element, and the distal end is capable of being inserted into the opening. In some embodiments, the strain-sensing element comprises a metal foil. In some embodiments, the strain-sensing element comprises a piezoelectric material. In some embodiments, the method further comprises a tooth clip configured to attach the strain gage to the tooth. In some embodiments, a first portion of the strain-sensing element is coupled to the member, and a second portion of the strain-sensing element is coupled to the tooth clip. In some embodiments, the tooth clip comprises an arcuate element configured to clip to the tooth. In some embodiments, the tooth clip comprises a shape-memory alloy and/or a superelastic material. The tooth clip may comprise a nickel titanium alloy.

### BRIEF DESCRIPTION OF THE DRAWINGS

FIGURE 1 is a cross-section view schematically illustrating a root canal system of a tooth.
FIGURE 2 is a scanning electron microscope photograph of a dentinal surface within an apical area of a root canal system of a mature tooth and shows numerous dentinal tubules on the dentinal surface.
FIGURE 3 is a cross-section view schematically showing an example of a method for cleaning a root canal system of a tooth, in which a high-velocity jet is directed toward a dentinal surface through an opening in the crown of the tooth.
FIGURE 4 schematically illustrates an embodiment of an apparatus for detecting motion of material within a root of a tooth.
FIGURE 5 is a block diagram schematically illustrating an embodiment of a system for cleaning teeth with a liquid jet.
FIGURE 6A is a cross-section view schematically illustrating an embodiment of an apparatus for sensing acoustic energy from a tooth.
FIGURE 6B is a photograph of an embodiment of the apparatus depicted in FIGURE 6A.
FIGURE 7A is a graph showing acoustic power sensitivity (relative to maximum power) in decibels (dB) versus frequency in megahertz (MHz) for a single-element ultrasonic transducer that may be used in the apparatus of FIGURE 6A.
FIGURE 7B is a graph showing amplitude of a pulse waveform versus time in microseconds (µs) for a pulse emitted by the ultrasonic transducer referenced in FIGURE 7A.
FIGURE 8A is a graph schematically illustrating an example of a pulse-echo trace that may be detected by an acoustic transducer positioned near a tooth. The graph depicts amplitude (in Volts) of the pulse-echo signal versus time and schematically depicts transmitted pulses and reflected echoes.
FIGURE 8B is another example of a graph schematically illustrating an example of a pulse-echo trace. FIGURE 8B also shows amplitude versus time for an electronic triggering pulse that may be used to trigger a piezoelectric transducer to transmit an acoustic pulse.

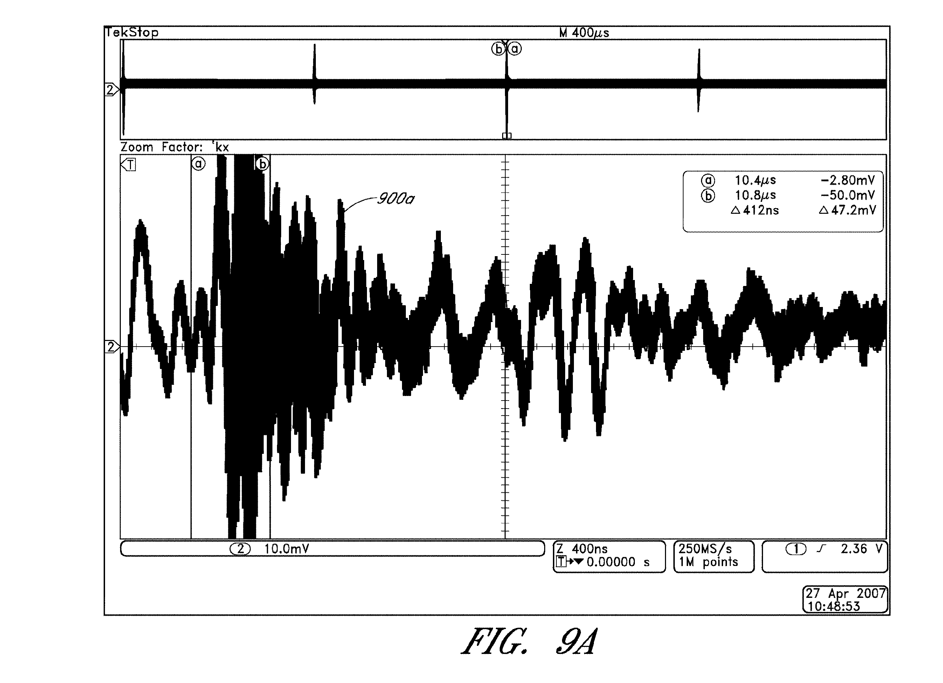

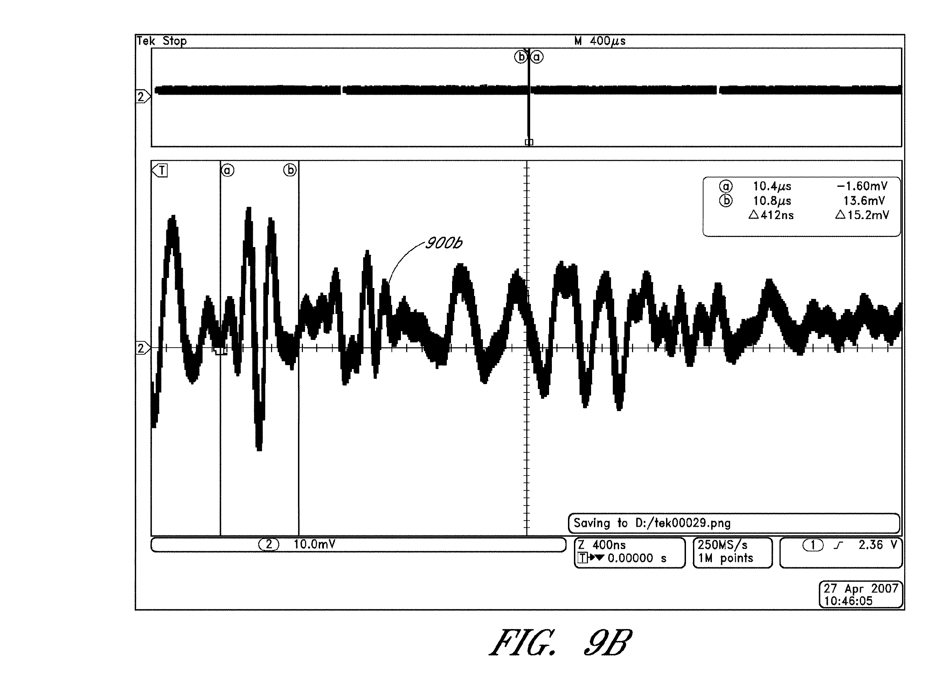

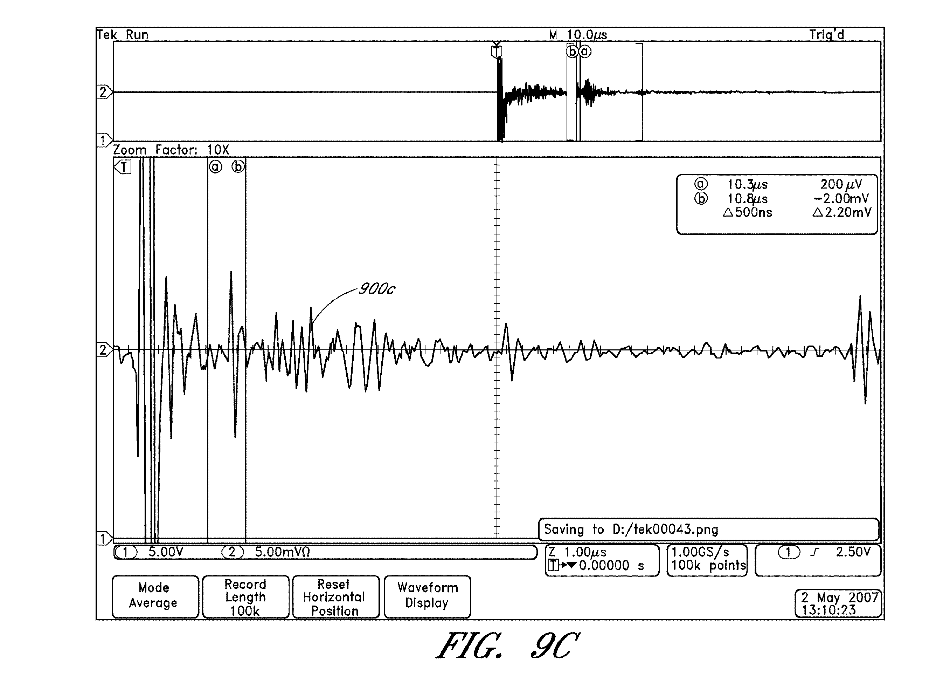

FIGURES 9A, 9B, and 9C are screen shots from a display device that show example pulse-echo traces detected by an acoustic transducer positioned adjacent a tooth having a flow of fluid passing therethrough. FIGURES 9A and 9B show amplitude (in Volts) versus time for echo signals propagating from the dentin-pulp chamber interface region. The screen shots in FIGURES 9A and 9B illustrate an envelope mode in which many reflected echoes are overlaid on each other. For comparison, FIGURE 9C shows a trace of a single echo. FIGURE 9A shows the results of an example in which the fluid was carbonated water, and FIGURES 9B and 9C show the results of an example in which the fluid was non-carbonated water.
FIGURE 10 schematically illustrates an example of the expected behavior, as a function of time, of the correlation of the acoustic echoes detected during root canal cleaning with the liquid jet.
FIGURES 11A and 11B are graphs depicting examples of the frequency sensitivity (FIG. 11A) and the directional sensitivity (FIG. 11B) of an embodiment of a hydrophone used to detect high frequency acoustic energy.
FIGURES 12A and 12B are graphs depicting examples of the frequency sensitivity (FIG. 12A) and the directional sensitivity (FIG. 12B) of an embodiment of a hydrophone usable to detect low frequency acoustic energy.
FIGURE 13 is a graph schematically illustrating an example of the rate of events (e.g., number of events per second) producing a high frequency acoustic signature versus time.
FIGURE 14 schematically illustrates two example power spectra that may be obtained by spectrally decomposing acoustic energy received from a tooth during cleaning with the liquid jet.
FIGURES 15A and 15B schematically illustrate a collimated liquid jet emitted by an embodiment of a handpiece and an embodiment of a spacer that may be used to adjust the working range of the jet.
FIGURES 15C-15E schematically illustrate embodiments of an aiming element that can be used with a dental handpiece.
FIGURE 15F schematically illustrates an embodiment of a dental handpiece configured to emit multiple liquid beams.
FIGURE 16 is a flow chart for an embodiment of a method of operation of a liquid jet apparatus used for endodontic procedures.
FIGURE 17 schematically illustrates an embodiment of a bimodal acoustic receiver capable of detecting acoustic energy in both a low-frequency range and a high-frequency range.
FIGURE 18A schematically illustrates an example of an acoustic coupling material interposed between an embodiment of an acoustic element and a tooth.
FIGURE 18B schematically illustrates an embodiment of an acoustic element configured to form an acoustic coupling tip in situ.
FIGURES 19A-19E schematically illustrate use of an embodiment of a strain gage to detect fluid flows in an opening in a tooth during an example dental procedure with a liquid jet.

### DETAILED DESCRIPTION OF PREFERRED EMBODIMENTS

The present disclosure describes apparatus and methods for sensing acoustic energy propagating from one or more regions in and/or near a tooth. The disclosed apparatus and methods advantageously may be used with root canal cleaning treatments, for example, to determine the efficacy of the treatment and/or to provide safety features that reduce risk of post-treatment complications. In some embodiments, the disclosed apparatus and methods are particularly effective when used with procedures using a high-velocity collimated beam of liquid to clean the root canal system. The high-velocity liquid beam may generate an acoustic wave that propagates through the tooth and detaches organic material from dentinal surfaces. The acoustic wave may cause acoustic cavitation effects (bubble formation and collapse, jet formation, acoustic streaming) that produce acoustic energy that propagates from the tooth. One or more acoustic elements may be used to detect the acoustic energy, and a processor may be used to analyze the detected acoustic energy for signatures representative of processes occurring in and/or near the tooth. For example, the acoustic signature of cavitation effects may be used for diagnostic and/or analytic purposes including, e.g., the determination of the progress of the root canal cleaning treatment and/or the presence or movement of material toward a periapical region of the tooth (e.g., near and/or through the apical opening). In some embodiments, acoustic transducers are used to transmit acoustic energy (e.g., ultrasound) toward a tooth and/or regions near the tooth. Acoustic receivers may be positioned to detect acoustic energy, which can be used for the diagnostic and/or analytic purposes described above. The detected acoustic energy may include a portion of the transmitted acoustic energy that propagates to the acoustic receiver and/or echoes of the transmitted energy.

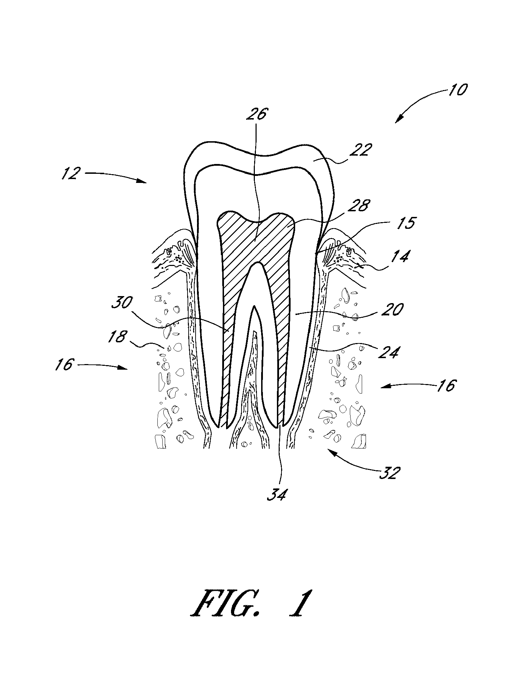

FIGURE 1 is a cross section schematically illustrating a typical human tooth 10, which comprises a crown 12 extending above the gum tissue 14 and at least one root 16 set into a bone socket within an alveolus of the jaw bone 18. Although the tooth 10 schematically depicted in FIGURE 1 is a molar, the apparatus and methods described herein may be used on any type of tooth such as an incisor, a canine, a bicuspid, or a molar. The hard tissue of the tooth 10 includes dentin 20 which provides the primary structure of the tooth 10, a very hard enamel layer 22 which covers the crown 12 to a cementoenamel (CE) junction 15, and cementum 24 which covers the dentin 20 of the tooth 10 within the boney socket.

A pulp cavity 26 is defined within the dentin 20. The pulp cavity 26 comprises a pulp chamber 28 in the crown 11 and a root canal space 30 extending toward an apex 32 of each root 16. The pulp cavity 26 contains dental pulp, which is a soft, vascular tissue comprising nerves, blood vessels, connective tissue, odontoblasts, and other tissue and cellular components. The pulp provides innervation and sustenance to the tooth through the odontoblastic lining of the pulp chamber 26 and the root canal space 30. Blood vessels and nerves enter/exit the root canal space 30 through a tiny opening, the apical foramen 34, near a tip of the apex 32 of the root 16.

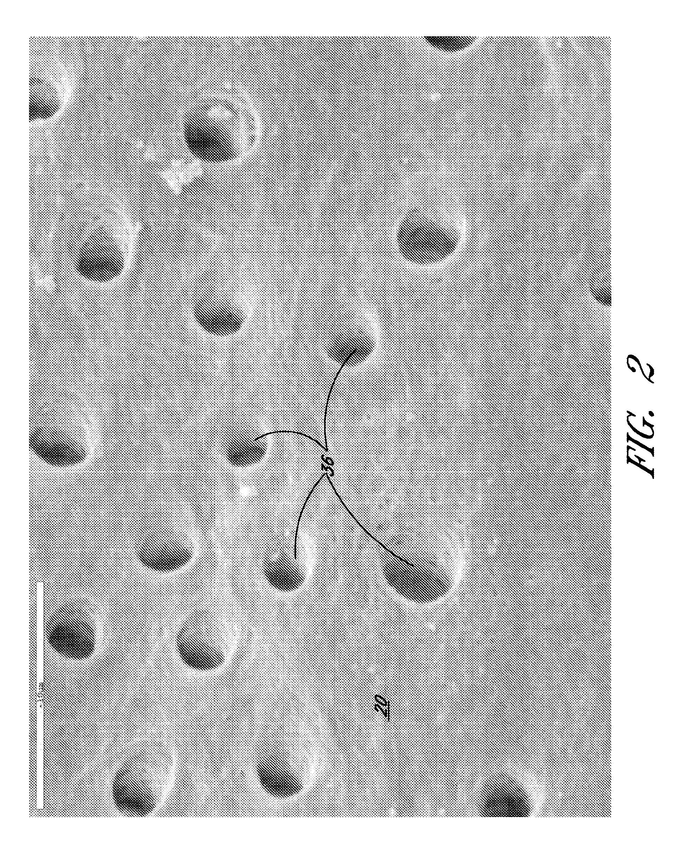

FIGURE 2 depicts a pulpal surface of the dentin 20. The dentin 20 comprises numerous, closely-packed, microscopic channels called dentinal tubules 36 that radiate outwards from the interior walls of the canal space 30 through the dentin 20 toward the exterior cementum 24 or enamel 22. The tubules 36 run substantially parallel to each other and have diameters in a range from about 1.0 to 3.0 microns. The density of the tubules 36 is about 5,000 - 10,000 per mm² near the apex 32 and increases to about 15,000 per mm² near the crown 12.

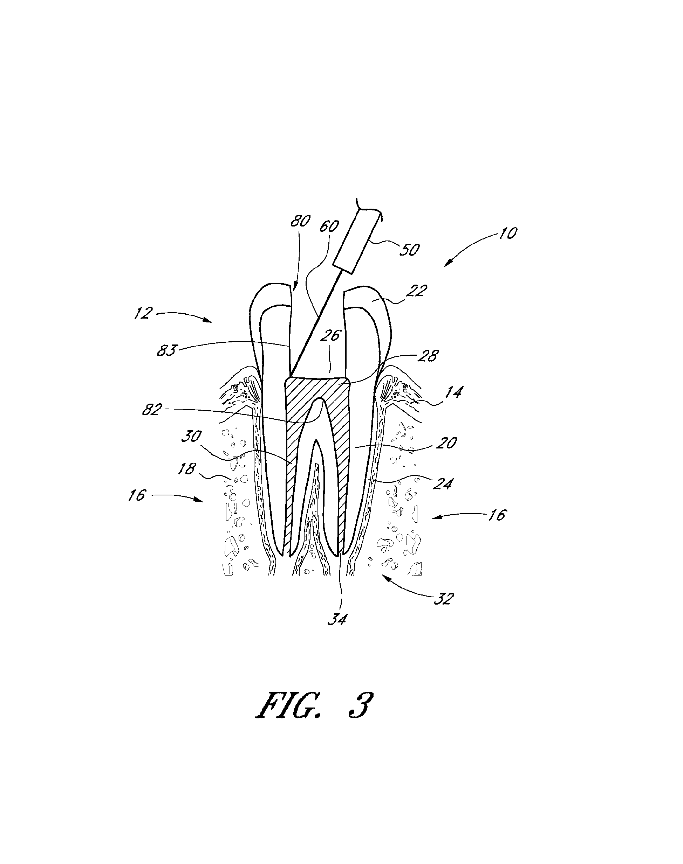

As discussed above, embodiments of the apparatus and methods disclosed herein advantageously may be used with various endodontic procedures, such as root canal treatments. A dental practitioner will recognize that the root canal system of the tooth 10 may be cleaned using any of a variety of endodontic modalities. Root canal cleaning may include, but is not limited to, at least partially detaching, excising, emulsifying, and/or removing organic (and/or inorganic) material from one or more portions of the pulp cavity 26 of the tooth 10 (including the pulp chamber 28 and/or canal space 30), and may include debridement. For example, a drill or grinding tool initially may be used to make an opening 80 in the tooth 10 (see FIG. 3). The opening 80 may extend through the enamel 22 and the dentin 20 to expose and provide access to pulp in the pulp cavity 26. The opening 80 may be made in a top portion of the crown 12 of the tooth 10 (as shown in FIG. 3) or in another portion such as a side of the crown 12 or in the root 16 below the line of the gum 14. The opening 80 may be sized and shaped as needed to provide suitable access to the pulp and/or some or all of the canal spaces 30. In some treatment methods, additional openings may be formed in the tooth 10 to provide further access to the pulp and/or to provide dental irrigation.

In some conventional root canal treatments, an endodontic file is inserted through the opening 80 to open the canal spaces 30 and remove organic material therefrom. The treatment may also remove from the canal spaces 30 inorganic material such as, e.g., dentinal filings caused by the filing process. Organic material (or organic matter) may include, but is not limited to, organic substances found in healthy or diseased root canal systems such as, for example, soft tissue, pulp, blood vessels, nerves, connective tissue, cellular matter, pus, and microorganisms, whether living, inflamed, infected, diseased, necrotic, or decomposed.

### Endodontic Apparatus and Methods Using Liquid Jets

An effective method for cleaning the root canal system is depicted in FIGURE 3, which schematically illustrates a high velocity collimated jet 60 of liquid (e.g., water) directed through the opening 80 toward a dentinal surface 83 of the tooth 10. Impact of the jet 60 causes couples kinetic energy from the collimated jet 60 into acoustic energy that propagates from the impact site through the entire tooth 10, including the root canal system. The acoustic energy is effective at detaching substantially all organic material in the root canal system from surrounding dentinal walls. The acoustic energy can detach organic material at locations in the tooth 10 that are remote from the impact site of the jet 60. In many embodiments, the detached organic material can be flushed from the root canal using irrigation fluid. The irrigation fluid may come from the high-velocity jet 60 and/or a source of low-velocity fluid.

The liquid jet 60 may be directed from a handpiece 50 that can be manipulated within a patient's mouth by a dental practitioner. In some embodiments, the liquid jet 60 is generated by a high pressure compressor system or a pump system. Further details of apparatus and methods for generating the high velocity jet 60 and using the jet 60 to clean root canal systems are found in U.S. Patent Application No. 11/737,710, filed April 19, 2007, entitled "APPARATUS AND METHODS FOR TREATING ROOT CANALS OF TEETH," which is hereby incorporated by reference herein in its entirety.

Following cleaning of the root canal system, the canal spaces 30 may be filled with a filling material and the tooth 10 restored. The filling material may comprise a thermoplastic material (such as gutta-percha). In some methods, hydrophobic and/or hydrophilic filling materials are used including, for example, the materials described in U.S. Patent Application No. 11/752,812, filed May 23, 2007, entitled "ROOT CANAL FILLING MATERIALS AND METHODS," which is hereby incorporated by reference herein in its entirety.

Some root canal treatments may suffer from possible disadvantages. For example, during treatment with an endodontic file, organic material and dentinal filings may be forced through the apical foramen 34 and into soft tissue surrounding the apex 32, possibly leading to complications such as infections. Also, a distal end of the file may pass through the foramen 34, leading to possible trauma. In cleaning methods utilizing the liquid jet 60, damage to soft tissue near the apex 32 of the root 16 may occur if the jet 60 is aimed directly down a root canal space 30 and the jet 60 impacts the periapical regions of the root 16 with sufficient force. Soft tissue damage may occur if there is incomplete apex formation of a root canal space 30 and the jet 60 sufficiently impacts the apex region. Additionally, during the canal filling process, filling material may migrate (or be forced) through the apical foramen 34 into the soft tissue near the apex 32. For example, in vertical and/or horizontal condensation of gutta percha, the gutta percha may be forced through the apical foramen 34 into periapical tissues.

Accordingly, it may be advantageous in certain techniques to detect the presence and/or the movement of material at periapical regions of the tooth 10 before such material passes through the apical foramen 34 and leads to possible complications. For example, in various embodiments of the disclosed apparatus and methods, the dental practitioner is alerted (e.g., by an audile, visible, and/or tactile signal) when material is detected near the apex 32 and/or detected to be moving toward the foramen 34. Upon receiving the alert, the practitioner beneficially can stop the treatment before causing potential damage. In other embodiments, the disclosed apparatus may detect the presence of the liquid jet 60 near the apex 32 and provide a signal to shut-off (or substantially reduce the energy of) the collimated jet 60. Therefore, the disclosed apparatus and methods advantageously may be used to increase the safety of a wide range of endodontic treatment methods.

### Acoustic Sensing Apparatus and Methods

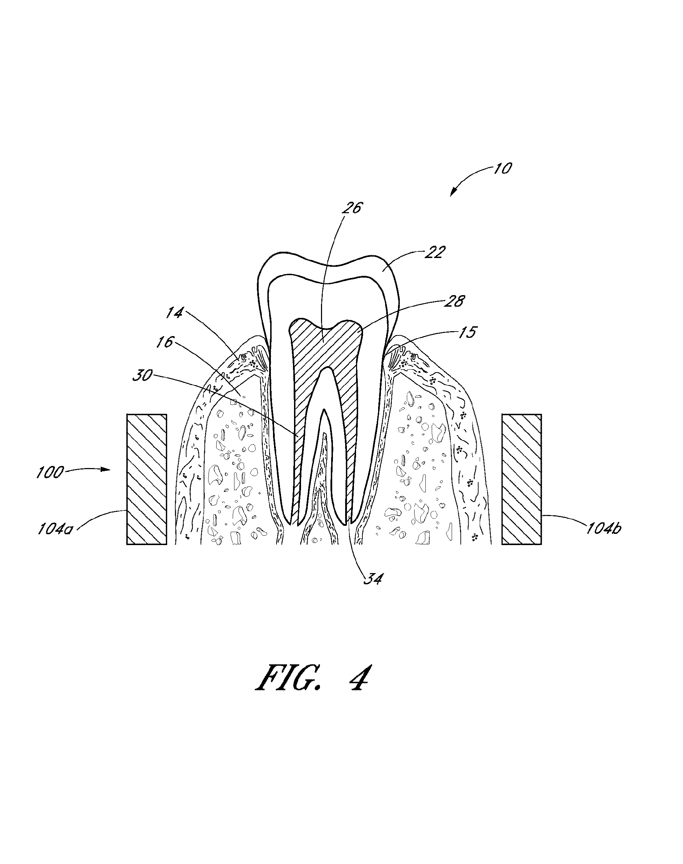

FIGURE 4 schematically illustrates an embodiment of an acoustic apparatus 100 that may be used in a variety of endodontic applications. For example, the apparatus 100 may be used for detecting presence and/or motion of material within (and/or near) a root 16 of a tooth 10. The apparatus 100 comprises acoustic elements 104a and 104b. In some embodiments, the acoustic element 104a comprises an acoustic transmitter that transmits acoustic energy toward the tooth 10 (and/or toward regions near the tooth 10), and the acoustic element 104b comprises an acoustic receiver 104b positioned to receive acoustic energy propagating from the tooth (and/or nearby regions). The received acoustic energy may include a portion of the transmitted acoustic energy that propagates along an acoustic path from the element 104a to the element 104b. The acoustic path may comprise a substantially straight line path and/or a path from the element 104 to a structure and/or material that redirects the acoustic energy toward the element 104b (e.g., by reflection, refraction, scattering, etc.). In another embodiment, either or both of the acoustic elements 104a, 104b may comprise an acoustic transceiver that can both transmit and receive acoustic energy. For example, in certain embodiments, the acoustic element may comprise a piezoelectric transducer having one or more piezoelectric crystals mounted on a substrate. A skilled artisan will recognize that although FIGURE 4 depicts two acoustic elements 104a and 104b, a different number of acoustic elements (transmitters and/or receivers) can be used in other embodiments. For example, the number of acoustic elements may be 1, 2, 3, 4, 5, 6, 10, 20, or more.

In various implementations, the acoustic element 104a generates acoustic energy in a suitable frequency range including, for example, an audible range (e.g., less than about 20 kHz) and/or an ultrasonic range (e.g., above about 20 kHz). In some embodiments, the frequency range includes megasonic frequencies above about 1 MHz such as, for example, a range from about 250 kHz to about 25 MHz. Other frequency ranges are possible, such as frequencies up to about 1 GHz. In various embodiments, the acoustic energy generated by the transmitter element 104a may be continuous-wave, pulsed, or a combination of continuous-wave and pulsed.

In some methods, the transmitter element 104a is placed adjacent to the tooth 10 under treatment, and the receiver element 104b is placed on the side of the tooth 10 opposite the transmitter element 104a. For example, the transmitter element 104a and the receiver element 104b may be positioned near the tooth 10 in a manner similar to well-known methods for positioning a dental x-ray slide. In some embodiments, the elements 104a and 104b are spatially fixed relative to the tooth 10 being treated, for example, by clamping to adjacent teeth or any other suitable fixation technique. The transmitter element 104a may be positioned on the lingual side or the buccal side of the alveolus of the tooth 10, with the receiver element 104b positioned on the opposing buccal or lingual side, respectively. In certain preferred embodiments, the transmitter element 104a is positioned to transmit acoustic energy through periapical regions of the tooth 10. In other embodiments, the acoustic energy may be transmitted through other portions of the tooth 10 (e.g., the canal spaces 30, the pulp chamber 28, etc.) or may be transmitted through substantially all the tooth 10.

In some implementations, the apparatus 100 operates by generating a transmitted acoustic beam with the transmitter element 104a and detecting a portion of the transmitted beam that propagates to the receiving element 104b. The receiving element 104b produces a signal in response to the detected acoustic energy of the beam. The apparatus 100 may include a general- or special-purpose computer configured to implement one or more known techniques for analyzing signals detected by the receiver element 104b. For example, the techniques may include analysis of phase shift and/or Doppler shift of the frequencies in the beam and/or analysis of spatial shift in the speckle pattern resulting from interference of energy in the acoustic beam. Spectral and/or wavelet analysis methods may be used. For example, the relative amplitude, phase, and amount of attenuation of spectral modes (and/or wavelets) may be detected and analyzed. Acoustic techniques may be used to measure reflection, transmission, impedance, and/or attenuation coefficients for the signal and/or its spectral modes (and/or wavelets). In some implementations, the detected acoustic energy is analyzed for the excitation of resonant frequencies. For example, the acoustic Helmholtz criterion may be used to related a resonant frequency to properties (e.g., volume, depth, height, width, etc.) of bores, chambers, canals, cracks, and so forth in the tooth. The decay of energy in a resonant acoustic mode (resonant ring-down) may be analyzed to determine attenuation coefficients in the tooth, as well as the presence of cracks and structural irregularities that increase the rate of the ring-down.

In some methods, the transmitter 104a generates a sequence of acoustic beams over a time period, and the receiver 104b produces a corresponding sequence of signals. The computer may process the signals independently or in combination. For example, in some implementations, the computer uses cross-correlation techniques to determine changes between portions of signals received at different times. In other implementations, other signal processing techniques are used. Accordingly, by suitably analyzing the acoustic energy detected by the receiver 104b, the apparatus 100 may calculate, for example, movement of material within the tooth 10, and in particular embodiments, movement near the apical foramen 34.

Thus, the apparatus 100 may be used to detect movement of material (including organic material, canal filling material, a portion of the endodontic file, and/or liquid from the jet 60) near the apical foramen 34. If movement of material is detected near the foramen 34, the apparatus 100 can produce a suitable response such as, for example, alerting the dental practitioner or shutting off the liquid jet 60.

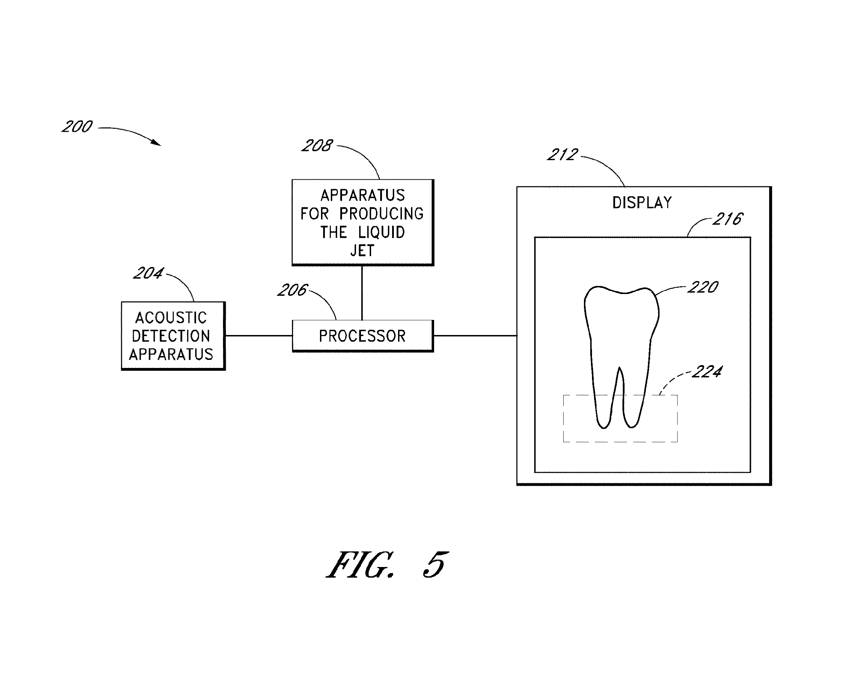

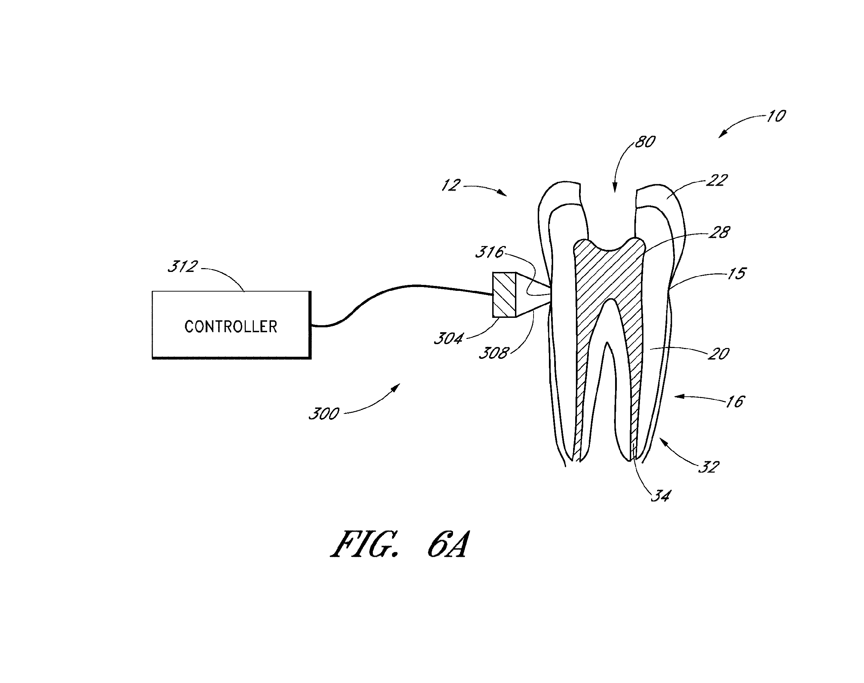

FIGURE 5 is a block diagram schematically illustrating an embodiment of a system 200 for cleaning teeth with a liquid jet. The system 200 includes acoustic sensing capability. The system 200 comprises an acoustic detection apparatus 204, a processor 206, an apparatus 208 for producing the liquid jet, and a display 212. The acoustic detection apparatus 204 may comprise any embodiments of the apparatus 100 described with reference to FIGURE 4 and/or any embodiments of the apparatus 300 described with reference to FIGURE 6A below. The processor 206 may comprise the general- or special-purpose computer described above for analyzing acoustic energy detected from a tooth (e.g., energy detected by the receiver 104b shown in FIG. 4). The jet-producing apparatus 208 may comprise a high pressure compressor system such as, for example, any of the systems described in the above-incorporated U.S. Patent Application No. 11/737,710, and/or in U.S. Patent No. 6,224,378, issued May 1, 2001, entitled "METHOD AND APPARATUS FOR DENTAL TREATMENT USING HIGH PRESSURE LIQUID JET," and/or in U.S. Patent No. 6,497,572, issued December 24, 2002, entitled "APPARATUS FOR DENTAL TREATMENT USING HIGH PRESSURE LIQUID JET," the entire disclosure of each of which is hereby incorporated by reference herein. The display 212 may comprise any suitable output device such as a cathode ray tube (CRT) monitor, a liquid crystal display (LCD), or any other suitable device. The display 212 may be configured to output an image 216 showing an actual (or schematic) image 220 of the tooth undergoing treatment. The image may also indicate a "target" 224 portion of the tooth 220.

In some embodiments, the acoustic detection apparatus 204 measures acoustic energy that propagates from the tooth under treatment. The apparatus 204 responsively communicates a suitable signal to the processor 206, which determines whether material is moving toward apical regions of the tooth. The measured acoustic energy may comprise ultrasonic energy as described above with reference to FIGURE 4. If material is detected moving toward the apical regions, the processor 206 automatically communicates a shut-off signal to the jet-producing apparatus 208, which shuts off flow of the high-velocity jet 60. In some embodiments, the jet-producing apparatus 208 (or another apparatus) continues to produce a lower velocity jet or flow of liquid (e.g., a stream of irrigating liquid) after the high-velocity jet 60 is shut off. Such embodiments may advantageously increase the safety of the liquid jet cleaning system 200 by terminating the high-velocity jet 60 before damage or trauma occurs to the tooth 10 and/or to tissue near the tooth 10. A further advantage is that the dental practitioner can concentrate on cleaning the root canal system of the patient without having to separately monitor the display 212 for movement of material toward the apices. Of course, in some embodiments, varying degrees of user-control over the shut-off signal is also provided so that the dental practitioner can stop the liquid jet 60 if the practitioner observes (on the display 212 or otherwise) undesired movement near the apices.

In some embodiments, the processor 206 generates the image 216 to be output on the display 212. In some preferred embodiments, the processor 206 operates under software instructions that allow the dental practitioner to "target" desired spatial locations of the tooth 220 (such as the apices as shown in FIG. 5) by designating a targeted region 224 of the image 216. For example, the spatial locations may be selected by positioning the target 224 (e.g., a "box" or other geometric figure illustrated in dotted lines in FIG. 5) around portions of the tooth 220. By designating such a target area, the processor 206 can operate to detect movement only in the corresponding locations in the tooth under treatment. An advantage of such embodiments is that by targeting desired locations of the tooth (e.g., the apices), the possibility of detecting movement of material at locations other than the target, which may generate an unwanted shut-off signal, is substantially reduced.

In some embodiments, the processor 206 is included in the jet-producing apparatus 208 and is not a separate element of the apparatus 200. In some embodiments, the processor 206 utilizes software instructions to determine whether movement is occurring at a target location (e.g., at the apices) and generates an appropriate shut-off signal in response to detected motion. In such embodiments, display of the image 216 is optional, because jet shut-off is determined automatically by the software instructions of the processor 206. Accordingly, the display 212 is not used in some embodiments. The shut-off signal may cause the jet-producing apparatus 206 to terminate the liquid jet 60. In some embodiments, the jet 60 is not completely stopped, but the speed of the jet 60 is reduced to a value that will not disrupt tissue. For example, in response to the shutoff signal, the jet-producing apparatus 206 may switch from a high-speed flow mode to a lower-speed irrigation flow mode. The acoustic sensing apparatus 100 depicted in FIGURE 4 can be configured differently in other embodiments, as will be further described below, to provide different acoustic sensing capabilities.

Certain preferred embodiments of the apparatus 100 are particularly useful in combination with the high-velocity liquid jet cleaning methods described above with reference to FIGURE 3. When the liquid jet 60 is directed against the dentinal surface 83 of the tooth 10, the jet 60 impacts the dentin with a force that produces an acoustic wave in the tooth 10. Accordingly, the impact of the jet 60 couples energy into the tooth at the impact site. The acoustic wave may propagate throughout the tooth, including the root canal system. The acoustic wave cleans the root canal system of the tooth 10 effectively and rapidly (within seconds in some embodiments). A possible theory for the effectiveness of the cleaning is that the acoustic wave produces acoustic cavitation effects (e.g., cavitation bubbles, cavitation jets, and/or acoustic streaming) that disrupt and separate organic material in the canal spaces 30 from surrounding dentin. The effectiveness of the cleaning is shown in FIGURE 2, which is a scanning electron microscope photograph of a cleaned dentinal surface. FIGURE 2 shows that the jet cleaning process has substantially eliminated organic material from the dentinal tubules 36 to a depth of about 3 microns.

The acoustic wave caused by the jet 60 causes processes in the tooth that may generate acoustic energy having an acoustic signature. The acoustic signature can be detected and analyzed to determine information related to the processes occurring in the tooth under treatment. For example, cavitation-induced effects (such as formation and collapse of cavitation bubbles and generation of cavitation jets) may produce acoustic energy with frequency components in the mega-Hertz range. The acoustic energy can be measured and used to determine, for example, effectiveness of the cleaning treatment and/or whether liquid from the jet 60 is flowing toward the apical foramen 34.

Accordingly, in another implementation of the apparatus 100 depicted in FIGURE 4, each of the acoustic elements 104a and 104b functions as an acoustic receiver to detect the acoustic energy caused by the liquid jet cleaning process. The elements 104a, 104b are hydrophones in some embodiments. Although two elements 104a and 104b are depicted in FIGURE 4, this is not intended to be a limitation on the range of possible apparatus 100. For example, in some embodiments, a single acoustic element is used to receive the acoustic energy. In other embodiments, more than two acoustic elements are used, such as 3, 4, 5, 6, 7, 10, or more elements.

The acoustic elements 104a and 104b may positioned in the mouth in the manner described above with reference to FIGURE 4, e.g., by clamping to adjacent teeth. In certain embodiments, one or more acoustic elements have an acoustic sensitivity that depends on the direction from which acoustic energy is received. The acoustic sensitivity typically has a peak sensitivity in a particular direction (e.g., perpendicular to the element in some cases). In such embodiments, some or all of the acoustic elements advantageously may be oriented within the mouth so that the peak acoustic sensitivity is directed toward a desired location in the tooth 10. When suitably positioned and/or oriented, the acoustic elements 104a, 104b may be focused to scan, map, image, and/or listen for acoustic energy emanating from portions of the tooth such as the root canal spaces and/or the apical openings.

During the liquid jet cleaning process, acoustic energy produced by impact of the liquid jet 50 against the tooth 10 may be guided within the canal spaces 30 and may propagate toward the apical foramen 34 (e.g., the canal spaces 30 may act as a wave-guide for acoustic energy). Since the canal spaces 30 generally become narrower in cross-sectional area in the longitudinal direction toward the apical foramen 34, the acoustic energy guided within the canal spaces 30 may be intensified at the apical foramen 34. This intensified acoustic energy may be detected by the acoustic elements 104a and 104b before any liquid or other material passes through the apical foramen 34 during the liquid jet cleaning process. Accordingly, detection of the intensified acoustic energy may be used to determine when to terminate the liquid jet so as to reduce the likelihood that liquid (or other material) passes through the apical foramen 34. For example, in some implementations, the processor 206 communicates a shut-off signal to the jet-producing apparatus 208 to terminate the high-speed liquid jet 50, if the intensity of the detected acoustic energy exceeds a threshold value that is selected to indicate that physical movement of material through an apical opening 34 is imminent.

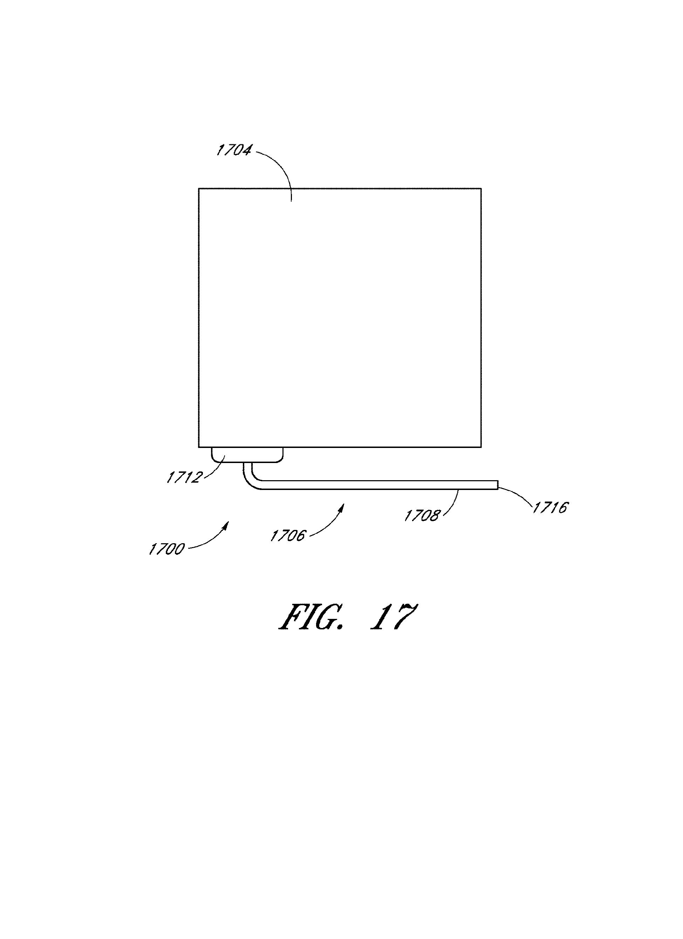

Embodiments of apparatus that detect intensified acoustic energy may provide several advantages. For example, the sensing apparatus 100 may utilize a single receiving element to detect the intensified acoustic energy (rather than the two elements 104a, 104b depicted in FIG. 4). Also, because the apparatus 100 listens for sound generated within the tooth 10, relatively simple acoustic receivers (e.g., hydrophones) may be used rather than more complicated and expensive acoustic transceivers, which both receive and transmit acoustic energy. In certain embodiments, the apparatus 100 uses one or more acoustic receivers that are capable of detecting both kilohertz and megahertz acoustic frequencies such as, for example, the bimodal acoustic receiver 1700 described with reference to FIGURE 17.

Embodiments of the apparatus described herein advantageously may be used with the liquid jet cleaning apparatus and methods to measure progress and/or efficacy of the treatment and/or to measure movement of material within the tooth during the treatment. As will be further described below, embodiments of some of these apparatus may be configured to operate in one or more acoustic sensing modes including, for example, a "pulse-echo" mode and/or a "passive listening" mode.

In certain embodiments of the pulse echo mode, an acoustic signal (e.g., one or more acoustic pulses) is propagated from an acoustic transmitter into the tooth under treatment. Echoes of the acoustic signal are detected by an acoustic receiver and analyzed by a processor. The acoustic receiver may be the same structure used to transmit the acoustic pulse, for example, a piezoelectric transducer capable of both transmitting and detecting acoustic energy. The echoes typically comprise acoustic energy from the transmitted acoustic pulse that is reflected, refracted, scattered, transmitted, or otherwise propagated to the acoustic receiver. For example, as is well known, a fraction of the acoustic energy incident on an interface between regions with differing acoustic impedances is reflected from the interface. In certain pulse-echo implementations, the transmitted acoustic pulse propagates into the tooth and reflects off such interfaces (e.g., an interface between dentin and pulp). The fraction of the reflected acoustic energy that propagates to the acoustic receiver may be detected and analyzed to provide information about properties of material at (or adjacent to) the interface.

In certain embodiments of the passive listening mode, one or more acoustic receivers are used to detect acoustic energy propagating from the tooth under treatment to the acoustic receivers. For example, the acoustic energy may be caused by cavitation-induced effects in the root canal system during the liquid jet cleaning process. In certain preferred embodiments of the passive listening mode, acoustic energy (e.g., acoustic pulses) is not transmitted into the tooth from an acoustic transmitter.

Embodiments of the apparatus described herein may operate in a pulse-echo mode or a passive listening mode. In some implementations, the apparatus may be operable in other sensing modes such as, for example, a combined mode in which acoustic energy is transmitted into the tooth under treatment and both reflected echoes and internally generated acoustic energy are detected and analyzed.

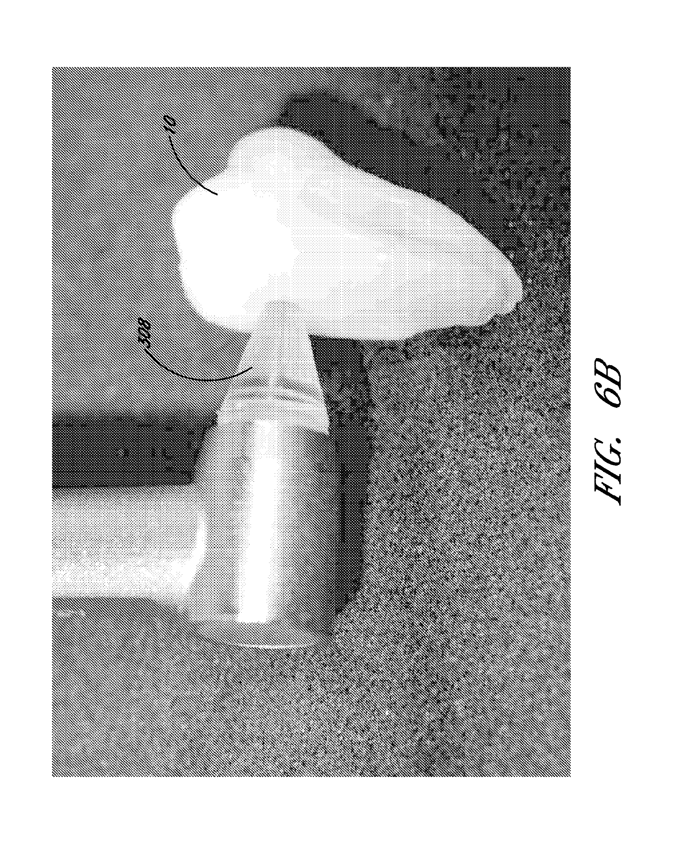

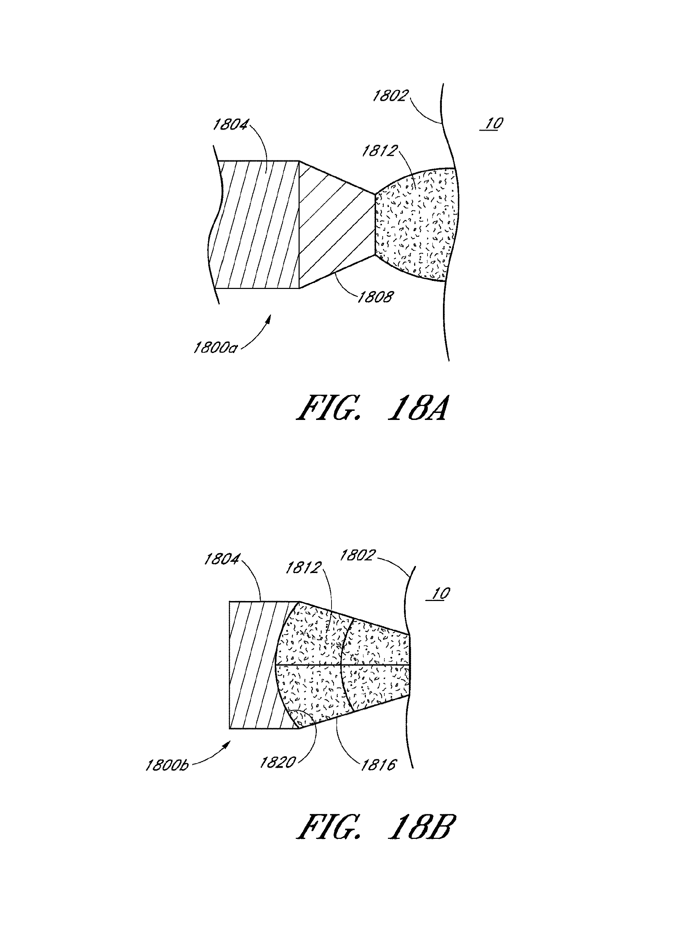

FIGURE 6A is a cross-section view schematically illustrating an embodiment of an apparatus 300 for sensing acoustic energy from the tooth 10. The apparatus 300 may be configured to operate in sensing modes including the pulse-echo mode, the passive listening mode, and/or the combined mode. The embodiment of the apparatus 300 depicted in FIGURE 6A comprises an acoustic transducer 304, an acoustic coupling tip 308, and a controller 312. The acoustic transducer 304 may comprise one or more single- and/or multiple-element transducers such as, for example, piezoelectric transducers. The transducer 304 may be operable to transmit and/or to receive acoustic energy. In passive listening embodiments, the acoustic transducer 304 may comprise one or more hydrophones that receive, but do not transmit, acoustic energy. The acoustic transducer 304 advantageously may be sized and shaped to fit within a patient's mouth. For example, in some embodiments, the transducer 304 is about 0.125 inches in diameter. In some embodiments, the acoustic transducer 304 is positioned at a distal end of a handpiece, which can be maneuvered within the patient's mouth by a dental practitioner. FIGURE 6B is a photograph of an embodiment of the apparatus 300 depicted in FIGURE 6A. The acoustic elements 1800a and 1800b described below with reference to FIGURES 18A and 18B may additionally or alternatively be used with the apparatus 300.

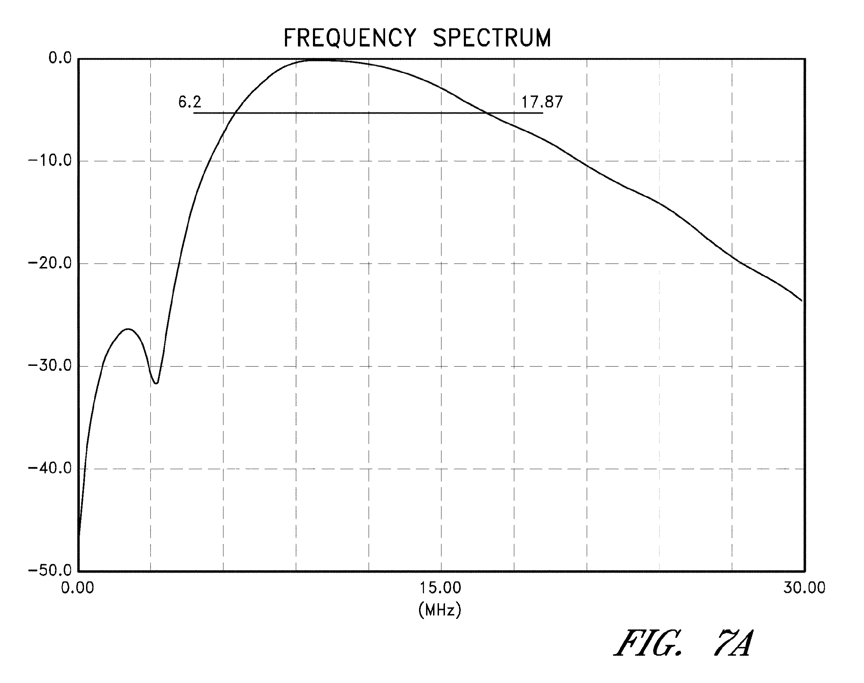

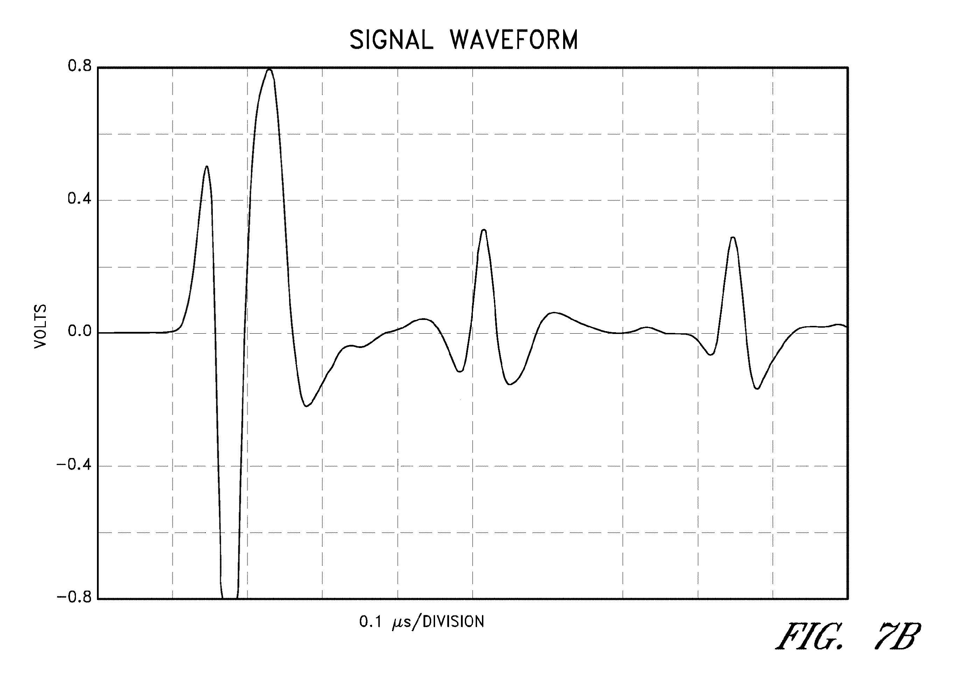

The acoustic transducer 304 provides acoustic sensing capability over a frequency range, which may include audible frequencies (below about 20 kHz) and/or ultrasonic frequencies (above about 20 kHz). The bimodal acoustic receiver 1700 (FIG. 17) may be used. FIGURE 7A is a graph showing acoustic power sensitivity (relative to maximum power) in decibels (dB) versus frequency in megahertz (MHz) for an example single-element ultrasonic transducer suitable for use with the apparatus 300. The maximum sensitivity of the transducer is at about 10 MHz, and the -5 dB frequency range is from about 6 MHz to about 18 MHz. FIGURE 7B is a graph showing amplitude versus time in microseconds (µs) for an example pulse transmitted from the single-element ultrasonic transducer described with reference to FIGURE 7A. The single-element transducer can transmit an acoustic pulse having an energy in a range from about 10 to about 100 microJoules (µJ). A pulse energy of about 25 µJ is used in some pulse-echo embodiments. In other embodiments, other pulse waveforms and other pulse energies are used. For example, the pulse waveform may be spectrally shaped or synthesized to provide an amplitude-modulated and/or frequency-modulated pulse shape such as, e.g., a chirped pulse or a coded pulse.

In some pulse-echo embodiments, the controller 312 is configured to communicate suitable control signals to the transducer 304 such as, for example, to energize the transducer 304 to generate an acoustic pulse (e.g., the pulse shown in FIG. 7B). The controller 312 also may receive signals indicative of the acoustic energy detected by the transducer 304. In certain embodiments, the controller 312 analyzes the detected acoustic energy, while in other embodiments, the analysis is performed by another processor or computer (e.g., the processor 206 shown in FIG. 5).

In an example pulse-echo application, the controller 312 energizes the transducer 304 to produce an acoustic pulse that is transmitted through the acoustic coupling tip 308 and into the tooth 10. The acoustic coupling tip 308 may be used as a relatively low-loss, impedance matching element between the acoustic transducer 304 and the tooth 10. In some embodiments, the tip 308 is fabricated from a polymer material such as polycarbonate. The acoustic coupling tip 308 may be configured as a signal delay line that provides a suitable time-delay between the transmitted pulse and reflected echoes from interfaces and structures in the tooth 10. The duration of the time-delay advantageously may be selected to reduce interference between the transmitted and reflected pulses. The shape of the tip 308 may be selected to act as a waveguide that focuses and/or collimates the acoustic energy transmitted from the transducer 304 so that a relatively high intensity acoustic pulse can be transmitted into the tooth 10. For example, FIGURE 6A schematically depicts an embodiment of the tip 308 having a generally frustoconical shape with a cross-section that narrows from the transducer 304 to an end 316 that is positionable adjacent to the tooth 10. To increase transmission of acoustic energy into the tooth 10, the tip 308 may be oriented so that the surface at the end 316 is substantially parallel to the surface of the tooth 10. Additionally, an ultrasonic coupling gel (or other suitable acoustic impedance matching substance) may be interposed between the end 316 and the tooth 10 to reduce undesired acoustic reflections between the tip 308 and the tooth 10.

In certain embodiments, the tip 308 is oriented so that a longitudinal axis of the tip 308 is substantially perpendicular to the tooth 10 (e.g., as shown in FIG. 6A). Acoustic energy transmitted into the tooth 10 propagates generally transverse to the pulp chamber 28 and the canal spaces 30 in such embodiments. In other embodiments, the longitudinal axis of the tip 308 is oriented at an angle to the tooth 10 in order to direct acoustic energy down a root 16 of the tooth 10. In these embodiments, the end 316 of the tip 308 may be angled or beveled so that the surface at the end 316 is substantially parallel to the surface of the tooth 10. A possible advantage of transmitting acoustic energy down the root 16 of the tooth 10 is that the Doppler shift of acoustic energy reflected from material moving longitudinally in the narrow canal spaces 30 may be detectable and may provide a diagnostic signal of motion near the apical foramen 34. In certain embodiments, the acoustic coupling tip 308 is disposed on the distal end of the handpiece 50 that directs the liquid jet 60 into the tooth 10. For example, the acoustic coupling tip 308 may be formed as a shroud that surrounds the nozzle that provides a collimated beam of liquid. Such embodiments may be advantageous, because a single handpiece can be used for the liquid jet 60 and the acoustic transducer 304.

The end 316 of the tip 308 may be positioned at any suitable location where it is desired for acoustic energy to be transmitted to and/or received from the tooth 10. It has been found that positioning the end 316 adjacent to a tooth surface near the CE junction 15 may be particularly effective in some methods, because acoustic energy transmitted into (or received from) the pulp chamber 28 does not pass through coronal enamel 22, thereby reducing acoustic attenuation and/or interfacial echoes that otherwise may be present. Also, the dentinal surfaces in the vicinity of the CE junction 15 are sufficiently smooth and regular that propagation of acoustic energy across dentinal surfaces (e.g., the dentin-pulp chamber interface or dentin-canal space interface) does not generate a significant amount of unwanted reflection, refraction, or scattering of the acoustic energy. Moreover, the CE junction 15 of the tooth under treatment is generally readily accessible to the dental practitioner. In some cases, the practitioner may slightly depress the gum 14 to access a suitable point near the CE junction 15.

The positioning of the end 316 of the tip 308 near the tooth 10 may be guided using information from the reflected echoes of the signal pulse. For example, in certain embodiments, pulse-echo waveforms are displayed on an output device (e.g., a monitor), and the tip 308 is maneuvered by a dental practitioner based on the observed waveforms. In some implementations, the dental practitioner may position or orient the tip 308 (and/or the end 316) to achieve an increased or maximal amplitude of a desired echo such as, for example, an echo from an interface between the dentin 20 and the pulp chamber 28 or canal space 30. Optimal positioning of the end 316 of the tip 308 may depend on the frequency range of the acoustic energy. For example, during acoustic sensing with 10 MHz acoustic energy, the optimal position of the end 316 of the tip 308 may be about 1.5 mm below the CE junction 15, whereas during acoustic sensing with 20 MHz energy, the optimal position may be about 3 mm below the CE junction 15. In some embodiments, the tip 308 is clamped (or otherwise fixed) when a desired or optimal position and/or orientation have been achieved.

In certain embodiments of pulse-echo apparatus and methods, the controller 312 causes a sequence of acoustic pulses to be transmitted into a tooth under treatment. As is well known, a transmitted pulse reflects from surfaces, interfaces, structures, and/or materials in the tooth 10 where there is an acoustic impedance mismatch. The reflected acoustic energy (e.g., echoes of the transmitted pulse) may be detected by the transducer 304 and communicated to the controller 312 for analysis. Although the present disclosure describes energy propagated to the transducer 304 as having been reflected from an interface, this description is for convenience of presentation only and is not intended to be a limitation on how acoustic energy can propagate. It is well recognized that acoustic energy can experience a wide variety of physical interactions while the acoustic energy propagates in matter. For example, acoustic energy may be reflected, refracted, scattered, transmitted, phase shifted, Doppler shifted, constructively and/or destructively interfered with, and so forth. Accordingly, it is recognized that the acoustic energy detected by the transducer 304 may have undergone one or more such physical interactions before detection.

As described above, FIGURE 7B shows an example waveform of a transmitted acoustic pulse in some embodiments. An acoustic pulse can have a bandwidth, which may be in range from about 1 MHz to about 25 MHz in some embodiments. Additionally, a sequence of acoustic pulses may be transmitted into the tooth under treatment at a pulse repetition rate, which is about 1 kHz in certain embodiments. The repetition rate may be selected so that a successive pulse in the sequence is not transmitted until substantially all the echoes of the preceding pulse have been received by the transducer 304.

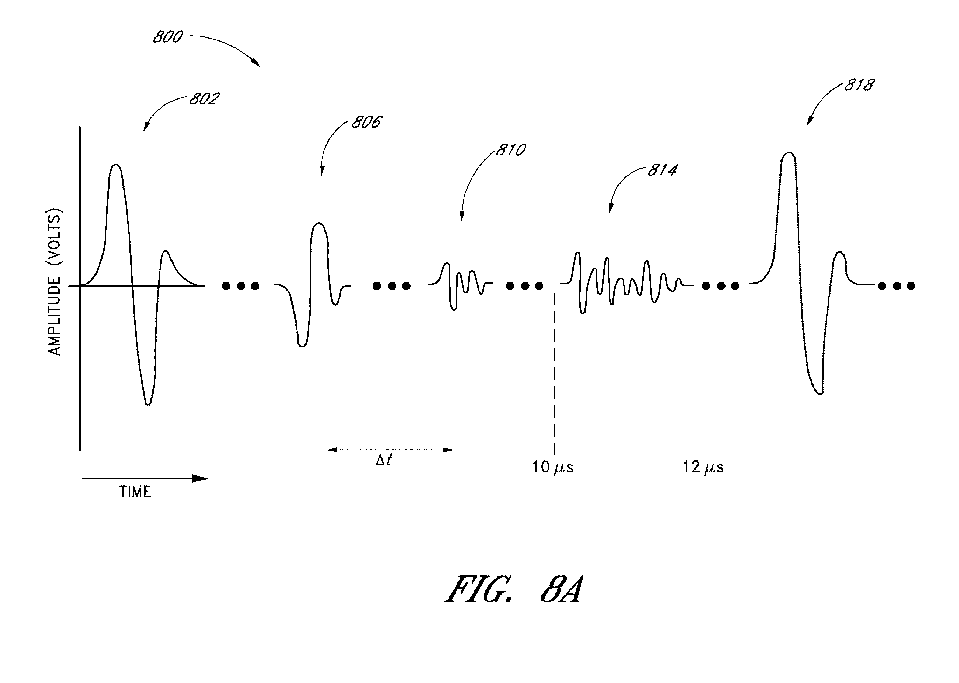

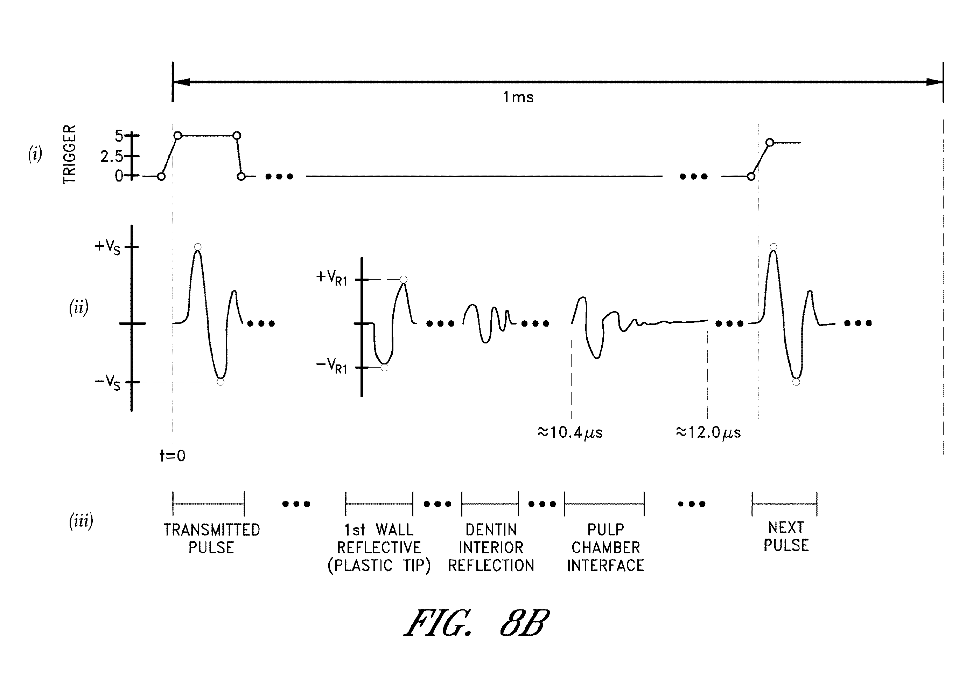

An example of a pulse-echo trace 800 that may be obtained using the apparatus 300 depicted in FIGURE 6A is schematically illustrated in FIGURE 8A, which depicts amplitude of an example signal detected by the transducer 304 versus time. The example pulse-echo trace 800 shown in FIGURE 8A includes a transmitted pulse 802 and reflected echoes 806, 810, and 814. In this example, a temporal sequence of pulses is transmitted into the tooth, and FIGURE 8A schematically illustrates pulse 818 as the next pulse transmitted after the pulse 802. The time duration between the transmitted pulses 802 and 818 is inversely related to the pulse repetition rate and is about 1 ms in some embodiments. The transmitted pulses 802, 818 may have substantially the same pulse waveform (e.g., as depicted in FIG. 8A) or may have different waveforms. A skilled artisan will recognize that the depicted waveforms of the transmitted pulses 802 and 818 are examples and are not intended to limit the type of pulses that may be used with embodiments of the apparatus 300. FIGURE 8B is another example of a graph schematically illustrating an example of a pulse-echo trace over a time period of about 1 ms. In line (i), FIGURE 8B shows amplitude versus time for an electronic triggering voltage that may be used to trigger a piezoelectric transducer to transmit an acoustic pulse. The triggering pulse may be communicated to the acoustic transducer 304 by the controller 312 or another suitable signal generator. In line (ii), FIGURE 8B shows voltages of the transmitted pulse and the reflected echoes in the pulse-echo train are shown. In line (iii), FIGURE 8B identifies the nature of the signals detected in the pulse-echo trace.

After being generated by the transducer 304, the transmitted pulse 802 propagates along the acoustic coupling tip 308 and may be focused, collimated, and/or intensified by the shape of the tip 308. Upon reaching the end 316 of the tip 308, a fraction of the energy in the transmitted pulse 802 is reflected at the interface between the end 316 and the tooth 10. The reflected energy propagates back along the acoustic coupling tip 308, is detected by the transducer 304, and is depicted as the reflected pulse 806 in FIGURE 8A. In some cases, the reflected pulse 806 is 180 degrees out of phase with the transmitted pulse 802 (e.g., as shown in FIG. 8A). As described above, the amount of energy in the reflected pulse 806 may be reduced by using an acoustic coupling gel between the end 316 and the surface of the tooth 10.

The pulse 802 continues to propagate into the tooth 10 and through the dentin 20. Some of the acoustic energy of the pulse 802 may reflect off structures in the dentin 20 and propagate back to the transducer 304 where the echoes are detected. The pulse-echo trace 800 schematically depicts dentinal reflections as the pulse 810. The amplitude, shape, and temporal extent of the pulse 810 will depend on the structure of the particular tooth under treatment.

The pulse 802 continues to propagate into the tooth 10 and reflects from other interfaces, structures, and materials that provide an acoustic impedance mismatch. For example, the pulse 814 shown in the pulse-echo trace 800 schematically represents example echoes from the interface between the dentin 20 and the pulp chamber 28 and echoes from material in the pulp chamber 28 that is near the interface. The amplitude, shape, and duration of the pulse 814 will depend on the particular tooth under treatment. As schematically depicted in FIGURE 8A, the reflected pulse 814 is detected at the transducer 304 about 10 µs after transmission. The time of about 10 µs approximately represents the round trip travel time for the transmitted pulse 802 to leave the transducer 304 and the reflected echo pulse 814 to return to the transducer 304. The duration of the reflected pulse 814 may range from about 0.5 µs to about 2 µs in certain embodiments. The properties of the reflected pulse 814 may be used to determine information about the material near the dentin-pulp chamber interface such as, for example, the composition of the material (e.g., pulp, liquid from the jet, etc.), the presence of acoustic cavitation effects caused by the liquid jet (e.g., cavitation bubbles), etc.

The pulse-echo trace 800 depicted in FIGURE 8A also shows the next transmitted pulse 818, which advantageously may be generated after substantially all the reflected pulses 806-814 are detected by the transducer 304. The pulse repetition rate is in a range from about 10 Hz to about 100 kHz in various embodiments and is about 1 kHz in certain preferred embodiments.

The controller 312 may be configured to store some or all of the pulse-echo trace 800 in a storage medium such as, for example, internal and/or external memory. The controller 312 may also be configured to process the pulse-echo trace 800 to determine properties within the tooth 10. For example, in some embodiments, one or more reflected pulses corresponding to a first transmitted pulse are correlated with one or more reflected pulses corresponding to a second transmitted pulse (which need not be the pulse immediately following the first pulse). The degree of correlation may provide a measure of time variability (if present) between the reflected pulses corresponding to the first and the second transmitted pulses. The measured time variability may indicate, for example, that material had moved within the pulp chamber 28 or the canal spaces 30 between the times of transmission of the first and the second pulse or that time-dependent processes were occurring within the tooth between the transmission times. In other embodiments, the controller 312 correlates a first portion of the pulse-echo trace 800 with a second portion of the trace 800. The portions may be chosen to reduce processing load on the controller 312, to identify particular features of interest (e.g., cavitation effects, movement, etc.), or for other suitable reasons. In other embodiments, other signal processing techniques may be used including, for example, time and/or frequency domain analysis techniques such as, e.g., autocorrelation, spectral decomposition (e.g., Fourier transforms), wavelets, filtering, etc. Analysis may be performed on analog and/or digital signals.

Embodiments of the apparatus 300 shown in FIGURES 6A and 6B may be used to determine the thickness of the dentin 20 in a tooth 10. The end 316 of the acoustic coupling tip 308 is placed against the outer surface of the tooth 10. An acoustic pulse is transmitted into the tooth 10, and reflected echoes indicative of the outer surface of the tooth 10 and a surface at the dentin-pulp chamber boundary are measured. For example, with reference to the pulse-echo trace 800 schematically depicted in FIGURE 8A, the pulse 806 corresponds to the reflected echo from the outer surface of the tooth 10, and the pulse 810 corresponds to the reflected echo from the inner surface at the dentin-pulp chamber boundary. The controller 312 can detect these echoes and calculate the time difference Δt between these two echoes. This time difference Δt is the round trip acoustic travel time between the outer surface and the inner surface. The thickness of the dentin may be estimated by multiplying one-half the time difference Δt by an estimated or measured speed of sound in dentin. The speed of sound in dentin may depend on whether the dentinal thickness is being measured on the buccal or lingual side of the tooth 10. In some embodiments, the speed of sound in dentin is estimated to be about 3000 m/s. By performing such pulse-echo measurements at different times (e.g., at different patient visits), the change in dentinal thickness in the tooth between these times may be calculated. A measured change may be used to determine the progress of endodontic disease in the tooth.

In certain endodontic procedures, the surfaces of the canal spaces 30 may be altered during the procedure, for example, by filing the canal spaces 30 with an endodontic file. Embodiments of the apparatus 300 may be used to measure roughness of the dentinal surfaces. For example, surface roughness reduces the back-reflection of acoustic energy in an incident acoustic pulse, because some of the acoustic energy is scattered by surface irregularities. Accordingly, by measuring an amplitude of a reflected echo (e.g., the echo 810 from the dentin-pulp chamber boundary), the controller 312 may estimate the surface roughness. In some embodiments, change in the amplitude of the reflected echo is measured during an endodontic procedure to determine the change in the surface roughness.

As described above, during a root canal cleaning treatment using the high-velocity liquid jet, impact of the liquid jet against a dentinal surface causes acoustic cavitation throughout the root canal system. The acoustic cavitation can include effects such as cavitation bubble formation and collapse, impingement of acoustic jets on dentinal surfaces, acoustic streaming, and/or entrainment of disrupted organic material. Acoustic signatures of the acoustic cavitation effects can be detected and analyzed with various embodiments of the apparatus 300. For example, the apparatus 300 can be operated in a pulse-echo mode to detect a pulse echo trace from the tooth under treatment. Additionally or alternatively, the apparatus 300 may be operated in a passive listening mode to detect acoustic energy propagating from within the tooth.

In one example embodiment, the apparatus 300 depicted in FIGURE 6A is used in a pulse-echo mode to determine properties of the interface between dentin 20 and the pulp chamber 28. FIGURES 9A, 9B, and 9C show graphs depicting pulse-echo traces 900a, 900b, and 900c, respectively, as measured by the acoustic element 304. In FIGURES 9A-9C, amplitude (in Volts) of the echo signal 900a-900c propagating from the dentin-pulp chamber interface region is plotted versus time. The figures are screenshots from an output device operably connected to the apparatus 300. The screenshots in FIGURES 9A and 9B show an "envelope" display mode of the output device in which echoes detected during a 5 second sampling time are overlaid upon each other. The screenshots in FIGURES 9A and 9B have been zoomed in to show a portion of the echo signals having a duration of about 5 µs. The portion has been selected to illustrate the time-variability of the echoes propagating from the dentin-pulp chamber interface. In the absence of measurable time variability in the material causing the reflections (e.g., material near the dentin-pulp chamber interface), each of the reflected echoes would overlap, and the graph shown in the screenshots would display a single line representing the constant shape of the reflected waveform. For example, FIGURE 9C shows a trace of a single echo, which displays as a narrow line 900c on the screenshot.

If there is measurable time-variability in the material near dentin-pulp chamber interface, successive echo signals will have slightly different waveform shapes and, when overlaid in the envelope display mode, will not precisely overlap the other signals. Therefore, the resulting display of the echo signals will appear, not as a single line (e.g., as in FIG. 9C), but rather as trace 900a having a "width." Accordingly, the amount of the "width" in the displayed trace 900a is a measure of the amount of time variability in the detected echoes, which is indicative of time-variability in the material causing the acoustic reflections. For example, the width of the waveform trace 900a shown in FIGURE 9A is greater than the width of the waveform trace 900b shown in FIGURE 9B, which indicates that material in regions near the dentin-pulp chamber interface experienced a greater degree of time variability under the conditions shown in FIGURE 9A than under the conditions shown in FIGURE 9B. The amount of time-variability in the echo signals (e.g., the "width" of the traces 900a, 900b in FIGS. 9A and 9B) may be quantified using a variety of signal processing methods. For example, in certain embodiments, the controller 312 correlates the echo signals (e.g., using auto- and/or cross-correlation techniques).

FIGURES 9A and 9B show example results using the apparatus 300 shown and described with reference to FIGURE 6A. The tooth 10 had been cleaned and was filled with a supply of fluid. The apical foramen 34 of the tooth 10 was enlarged slightly so that the fluid could smoothly flow through the tooth at a rate of about 1 ml/s, which is approximately the rate of fluid delivery in some high-velocity liquid jet systems. While the fluid was flowing through the tooth 10, the transducer 304 transmitted a sequence of acoustic pulses and measured the reflected echoes. As described above, FIGURES 9A and 9B are envelope mode displays of the echoes from regions in the tooth near the dentin-pulp chamber interface. To determine which echoes came from the dentin-pulp chamber interface region, the acoustic travel time from the transducer 304, to the interface, and back to the transducer 304 (where the echoes are detected) was estimated. The echo signals corresponding to this travel time (about 10 µs to about 12 µs) are displayed in FIGURES 9A and 9B as the traces 900a and 900b, respectively. The portion of the echoes shown between lines marked "a" and "b" is believed to be indicative of acoustic reflections from dentin-pulp chamber interface. The portion of the echoes following the line marked "b" is believed to be indicative of acoustic reflections from material beyond the dentin-pulp chamber interface and within the pulp chamber 28.

FIGURE 9A shows example results with the apparatus 300 shown in FIGURE 6A in which the fluid flowing through the tooth 10 was carbonated water (e.g., soda water). FIGURE 9B shows example results in which the fluid was non-carbonated water (e.g., tap water). Carbonated water contains substantially more bubbles (per unit volume) than non-carbonated water and was selected to represent conditions in a tooth undergoing acoustic cavitation during liquid-jet cleaning. The width of the echo traces 900a and 900b shown in FIGURES 9A and 9B demonstrate that the presence of bubbles in the carbonated water (see FIG. 9A) causes greater time-variability in the reflected echo signal than in the example with water having relatively fewer bubbles (see FIG. 9B). Accordingly, FIGURES 9A and 9B demonstrate that embodiments of the apparatus 300 may be used in a pulse-echo mode to detect at least the presence (and/or absence) of bubbles in material near the dentin-pulp chamber interface. The width of the traces 900a, 900b may provide a quantitative estimate of the bubble density in the canal spaces 30.

Therefore embodiments of the apparatus 300 advantageously may be used to detect the presence of acoustic cavitation-induced effects occurring during application of the high-velocity liquid jet 60. Moreover, because it is believed that the acoustic cavitation process occurs substantially simultaneously throughout the entire root canal system of the tooth 10 during the jet cleaning treatment, measurements of cavitation effects performed with a transducer positioned anywhere near the tooth 10 may be indicative of acoustic cavitation occurring throughout the entire tooth, including at locations remote from the transducer. For example, as shown in FIGURE 6A, positioning the transducer 304 near the C-E junction 15 may be particularly advantageous, because the C-E junction 15 is generally more accessible to the dental practitioner than regions toward the root. An additional advantage of positioning the transducer 304 near the C-E junction 15 is that the transmitted and reflected acoustic signals propagate through less intervening material than at positions near the root where there may be a substantial amount of intervening gum 14 and bone 18. Accordingly, acoustic signals transmitted and received at the C-E junction 15 will suffer less attenuation and less spurious acoustic reflections from intervening material as compared to acoustic signals transmitted and received near the periapical regions. Therefore, in some embodiments, the transducer 304 is positioned near the C-E junction 15 and is used to detect one or more acoustic signatures associated with the jet cleaning process. Some of the acoustic signatures may be indicative of the acoustic effects occurring in portions of the root canal system near to and/or remote from the position of the transducer 304.

In some embodiments, the apparatus 300 may be used to determine when root canal cleaning by the high-velocity jet is substantially complete and to shutoff the high-velocity jet 60. Such embodiments advantageously reduce the likelihood of damage to dentinal surfaces from impact of the jet 60 and reduce the likelihood that the jet 60 will unintentionally be directed down the canal space 30 toward the apical foramen 34. In some embodiments, after the high-velocity jet 60 is shut off, the jet apparatus 308 may continue to produce a low-velocity irrigation flow.

The correlation of the echo signals from the dentin-pulp chamber interface region is used to determine the progress of the liquid jet cleaning process in certain preferred embodiments. Before application of the high-velocity jet, there will be relatively few bubbles (or other cavitation-induced effects) within the root canal system, and the material in the pulp cavity 26 will reflect acoustic pulses in a relatively repeatable pattern. The resulting pulse-echo trace may appear similar to that shown in FIGURE 9B, and the correlation of the echo signals will be relatively high. However, when the high-velocity jet is applied to the tooth 10, acoustic cavitation effects (e.g., cavitation bubbles) will occur throughout substantially the entire root canal system, and the reflected acoustic pulses will exhibit a greater degree of variability. The resulting pulse-echo trace may appear similar to that shown in FIGURE 9A, and the correlation of the echo signals will be reduced as compared to the correlation before application of the jet 60. As the cleaning process approaches completion, acoustic cavitation effects are expected to decrease throughout the tooth 10. Liquid from the jet 60 may begin to flow within the canal space 30, and the pulse-echo trace may return to the appearance shown in FIGURE 9B. The correlation of the echo pulses will tend to increase, because the concentration of cavitation bubbles will tend to decrease as the cleaning process completes.

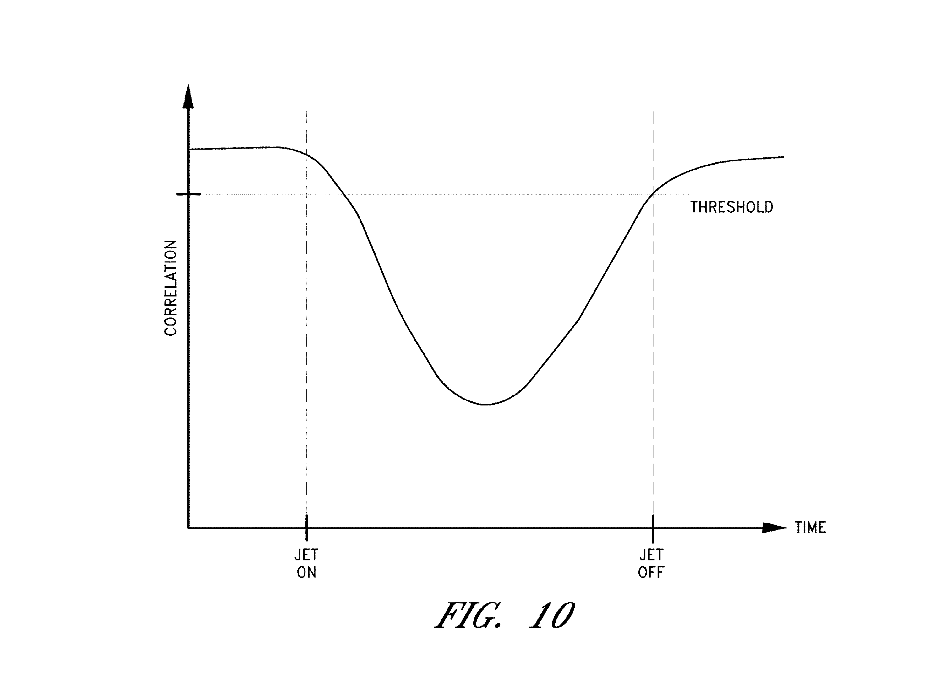

Accordingly, in some embodiments of the apparatus 300, the controller 312 monitors the correlation of the echo signals propagating from the dentin-pulp chamber interface region. FIGURE 10 schematically illustrates an example of the expected behavior, as a function of time, of the correlation of the echoes. Initially, the correlation is relatively high. The correlation decreases after the liquid jet 60 is actuated, because cavitation-induced effects begin to cause time-variability in the reflected echoes. As cleaning progresses and cavitation-induced effects reach a maximum, the correlation decreases to a minimum. The correlation then rises as cleaning is completed, and the concentration of cavitation bubbles decreases. In some embodiments, the apparatus 300 communicates a shutoff signal to the jet producing apparatus 208 when the correlation rises above a threshold (see FIG. 10). The threshold may depend on the type of tooth under treatment (e.g., molar, bicuspid, canine, incisor), the degree by which the canal spaces are filled with organic material, and other factors. Accordingly, by monitoring the correlation of the echoes, embodiments of the apparatus 300 may automatically produce a shutoff signal that terminates the high-velocity jet 60 when cleaning is substantially complete in the tooth under treatment. Such embodiments advantageously permit the liquid jet 60 to impact dentinal surfaces of the tooth for a time sufficient to provide effective cleaning and automatically terminate the jet 60 before unwanted damage to the dentin 20 can occur or unwanted liquid can impact the periapical regions of the tooth. Additionally, an effective treatment time for the particular tooth is determined automatically (e.g., based on the time it takes the correlation value to reach the threshold); hence, the dental practitioner does not need to make possibly error-prone estimates for the treatment time.

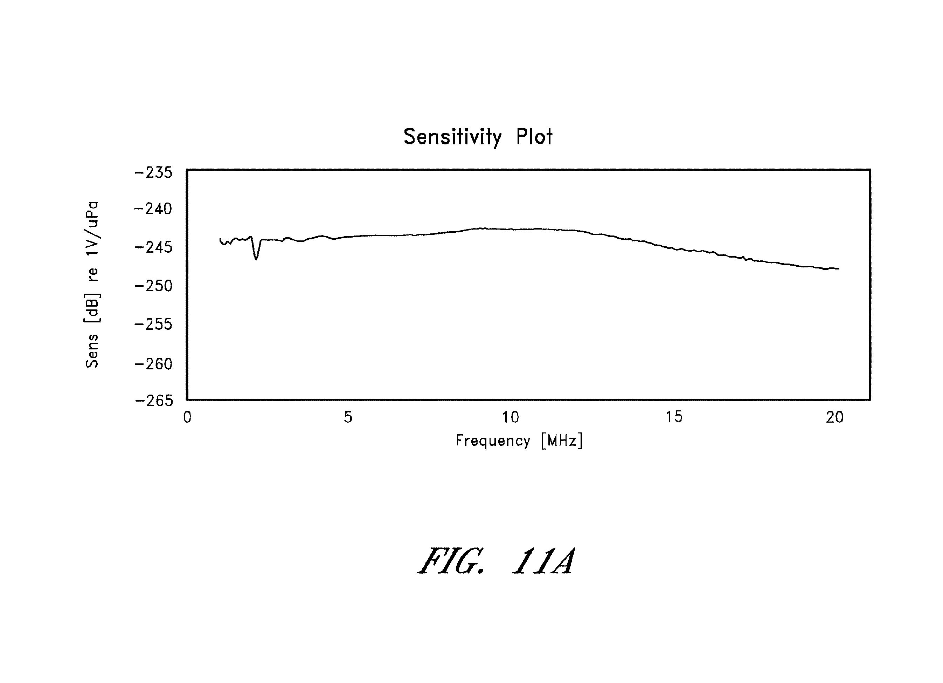

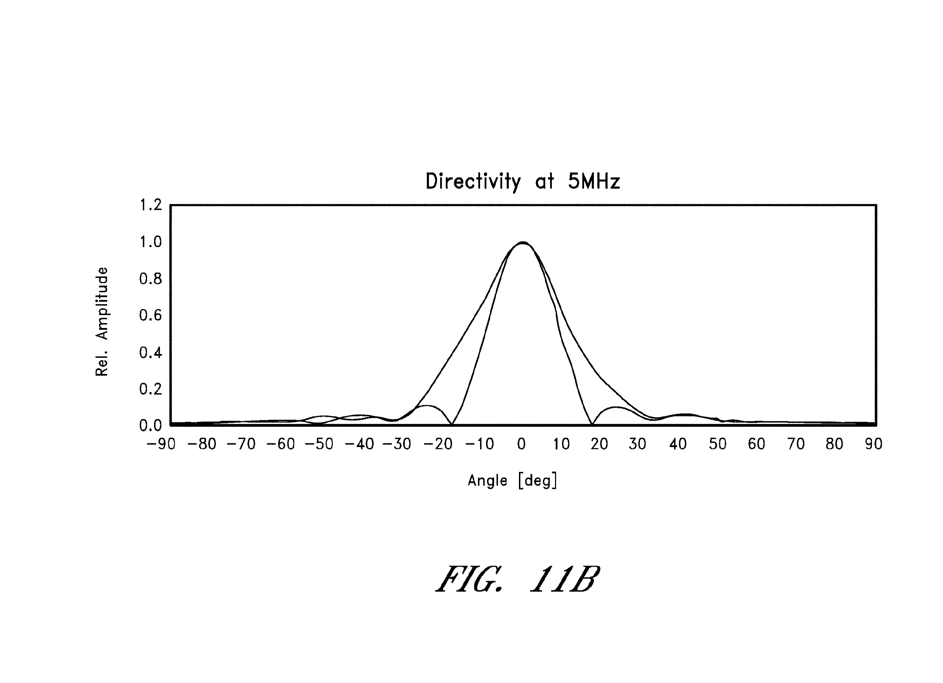

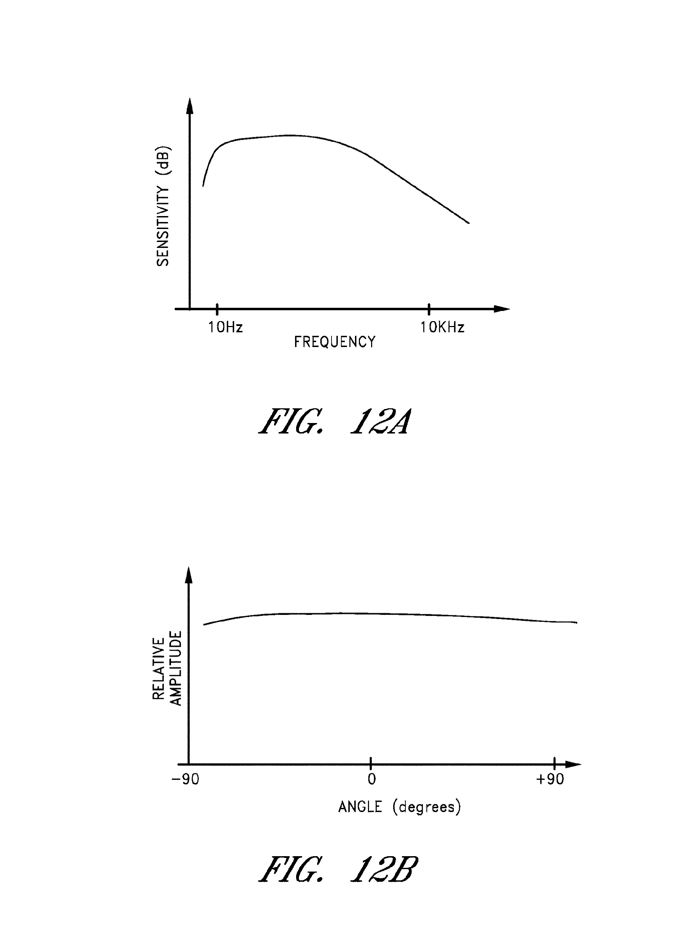

As described above, embodiments of the apparatus 300 may be operated in a passive listening mode in which one or more acoustic receivers are positioned near a tooth under treatment to detect acoustic energy propagating from the tooth. In some implementations, the transducer 304 may be positioned relative to the tooth as shown in FIGURES 6A and 6B. In some embodiments, the acoustic receivers can be hydrophones that detect acoustic energy but which do not transmit acoustic energy into the tooth. FIGURES 11A and 11B are graphs depicting the frequency sensitivity (FIG. 11A) and the directional sensitivity (FIG. 11B) of an embodiment of a hydrophone used to detect high frequency acoustic energy. The frequency sensitivity of the high-frequency hydrophone may be in a range from about 200 kHz to about 25 MHz. Higher frequencies may be used in other embodiments (e.g., up to about 1000 MHz). FIGURES 12A and 12B are graphs depicting the frequency sensitivity (FIG. 12A) and the directional sensitivity (FIG. 12B) of an embodiment of a hydrophone used to detect lower frequency acoustic energy (e.g., audible frequencies below about 20 kHz). The frequency range for this embodiment comprises frequencies from about 10 Hz to about 10 kHz. Acoustic receivers having different frequency sensitivities may be use, for example, with a range that includes higher frequencies (e.g., to about 200 kHz). The directional sensitivity of this embodiment of an audible-frequency hydrophone is relatively flat, e.g., the hydrophone is substantially omnidirectional. FIGURES 11A-12B are intended to be nonlimiting examples of the frequency and directional sensitivities of various hydrophones that may be used with the apparatus 300. In other embodiments of the apparatus 300, acoustic receivers (and/or transmitters) can have different sensitivities than shown in FIGURES 11A-12B. For example, in certain embodiments, the bimodal acoustic receiver 1700 shown and described with reference to FIGURE 17 is utilized.

The acoustic energy propagating from the tooth under treatment may comprise one or more acoustic signatures indicative of processes occurring within the tooth. In some embodiments, the acoustic signatures comprise energy responsive characteristics associated with the detected acoustic energy. The acoustic energy can be detected by an acoustic receiver and analyzed by a suitable processor (e.g., the controller 312). In certain preferred embodiments, the detected acoustic signatures are used to provide a shutoff for the liquid jet 60 when the cleaning treatment is substantially complete.

The high-velocity liquid jet cleaning process may cause various acoustic signatures. For example, a high-frequency acoustic signature (which may comprise megahertz frequencies) and a low-frequency acoustic signature (which may comprise audible frequencies) may provide information related to processes occurring during root canal cleaning with the jet. The acoustic signatures may be in response to energy coupled into the tooth 10, for example, by impact of the high-velocity liquid jet.

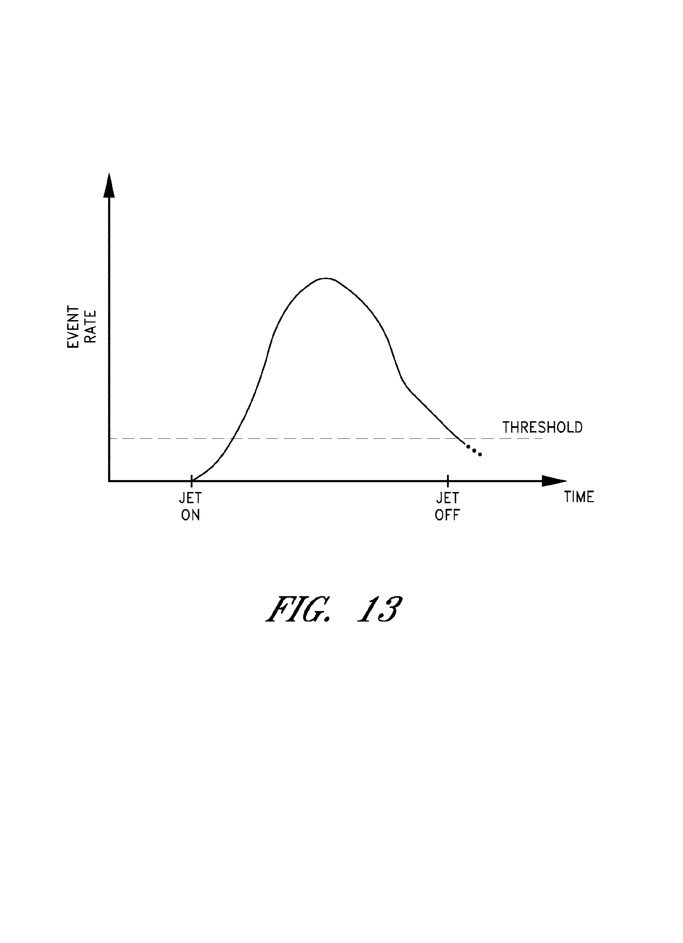

In certain cases, the high-frequency signature may be detected in a frequency range from about 200 kHz to about 25 MHz. The high frequency signature may also include higher frequencies such as, for example, to about 100 MHz and/or to about 1 GHz. The high frequency signature is believed to be representative of acoustic energy produced by events such as the formation and collapse of cavitation bubbles. The high frequency acoustic signature may also comprise events such as impingement of cavitation jets on dentinal surfaces. The events causing the high frequency signature generally may be short duration, transient, events occurring in the root canal system of the tooth. FIGURE 13 is a graph schematically illustrating the rate of events (e.g., number of events per second) producing the high frequency acoustic signature versus time. The acoustic signature of the events may be detected by the transducer 304 and/or other acoustic receivers positioned near the tooth under treatment. Before the liquid jet is actuated and impacts a dentinal surface of the tooth, the event rate is approximately zero. After the liquid jet is actuated and is used to clean the root canal system, the event rate causing the high frequency acoustic signature increases, because, for example, additional surface area in the root canal system becomes available for cavitation-induced effects. As root canal cleaning progresses towards completion, the rate of events causing the high frequency acoustic signature may reach a maximum and then decrease.

In certain embodiments, the apparatus 300 detects the high frequency acoustic energy propagating from the tooth, and the controller 312 determines the event rate. The event rate may be determined from the amplitude and/or intensity of the high frequency acoustic energy detected by the transducer 304. In some embodiments, the event rate is determined from a rate of change of the amplitude and/or intensity of the detected acoustic energy. In certain embodiments, the apparatus 300 analyzes the event rate (and/or other characteristics of the acoustic signature) and determines when the treatment is substantially complete. Based at least on this determination, the apparatus 300 may automatically communicate a shutoff signal to the high velocity jet producing apparatus (e.g., the apparatus 208 shown in FIG. 5). For example, as depicted in FIGURE 13, the shutoff signal may be communicated when the event rate decreases below a threshold. The threshold may be selected to ensure that substantially all the root canal system has been effectively cleaned.

In some cases, the event rate may be indicative of the number of acoustic cavitation bubbles forming (and collapsing) per second in the root canal system (e.g., in the tubules 36 and/or near the walls of the dentin 20). The cleaning process in some cases may be particularly effective after a threshold number of bubbles (or other cavitation effect) may have formed and collapsed near a given tubule 36 or near a given dentinal surface area (e.g., 1 square micron). For example, in certain cases, the threshold number may be 10, 100, 1000, 10,000, or some other number of bubbles. Accordingly, in some embodiments, the event rate threshold shown in FIGURE 13 may be selected to provide that the tooth under treatment has experienced the threshold number of bubbles (or other cavitation effects) sufficient to provide effective cleaning of substantially the entire root canal system. For example, the event rate threshold may be determined such that the cumulative event rate (e.g., the area under the curve depicted in FIG. 13) is sufficient for substantially all the surface area of the root canal system to have been cleaned by the threshold number of cavitation bubbles.

As discussed above, during treatment with the liquid jet, acoustic energy propagating from the tooth may exhibit a low frequency acoustic signature comprising frequencies in the audible frequency range (e.g., below about 20 kHz). In some cases, the frequency range comprises frequencies from about 10 Hz to about 10 kHz. In certain cases, the frequency range includes frequencies from about 500 Hz to about 5 kHz. In some embodiments of the apparatus 300, the transducer 300 used to detect the acoustic energy comprised a hydrophone having frequency and directional sensitivities shown in FIGURES 12A and 12B.

The low frequency acoustic energy may be representative of processes including, for example, filling and discharge of fluid from the canal spaces 30, resonance of acoustic oscillations of the canal spaces 30, and/or other energy responsive characteristics associated with the cleaning process. The low frequency acoustic energy may be caused by physical processes and structures with a larger spatial scale than the processes and structures responsible for the high frequency acoustic energy. Low frequency acoustic signatures may be indicative of the spatial dimensions and configuration of portions of the canal spaces 30 including, for example, the interior volume and/or geometry of the pulp chamber 28, canal space 30, etc.

A low frequency acoustic signature may include a Helmholtz resonant frequency of the tooth 10, including resonant frequencies of portions of the pulp chamber 28, canal spaces 30, etc. As is well known, the Helmholtz resonant frequency may be related to properties of the resonating chamber including, for example, volume, height, width, and/or depth of the chamber. In certain embodiments, one or more images of the internal structure of the tooth 10 is taken (e.g., a standard dental X-ray). Size information for one or more internal tooth chambers may be measured from the image (which may include one or more fiducial length markers). Acoustic models based on the measured size information can be used to calculate resonant frequencies of the tooth 10. In certain embodiments, the low frequency acoustic signature is used to determine a measured resonant frequency, and the acoustic models (and the one or more images) are used to reconstruct the size of the tooth chambers.

During the root canal cleaning process using the high velocity liquid jet, an interior volume of the root canal system may undergo at least partial filling and expulsion of liquid. The filling and expulsion may be periodic or quasiperiodic in some treatments and may generate detectable low frequency acoustic energy. For example, in some cleaning methods, a single hole in an occlusal surface of the tooth 10 (e.g., the opening 80) is used to inject the liquid jet beam 60 and to evacuate liquid and detached organic material from the canal space 30. The volume of the canal space 30 that is filled with liquid and/or organic material may fluctuate and/or oscillate with time. The rate of this fluctuation (and/or oscillation) may be determined from the size and geometry of the canal space 30. In some cases, a lower bound on the oscillation rate may be reached when the space 30 is substantially free of organic material and filled with liquid from the jet 60 at a substantially constant flow rate. Acoustic energy caused by the fluctuations and/or oscillations of the root canal system can be detected (e.g., by a hydrophone) and analyzed to determine when to shut off the liquid jet 60.

In some embodiments, the controller 312 of the apparatus 300 communicates a shutoff signal for the liquid jet 60 when a suitable low-frequency acoustic signature is detected. The acoustic signature may be indicative of an oscillation frequency (e.g., a Helmholtz resonant frequency) of a portion of the canal space 30. In some embodiments, the signature comprises a rate of change of an oscillation frequency decreasing below a threshold. In other embodiments, the signature comprises a change in a frequency band (e.g., a range of frequencies around an oscillation frequency), a change in amplitude and/or intensity of the low-frequency acoustic energy, and so forth.

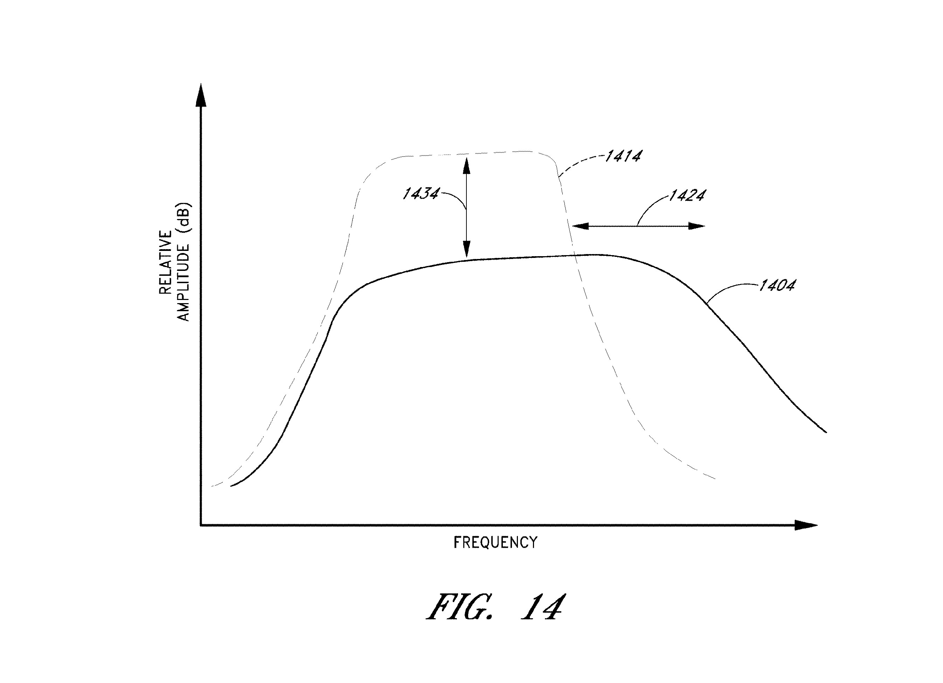

FIGURE 14 schematically illustrates two example power spectra 1404 and 1414 that may be obtained by spectrally decomposing the acoustic energy received from a tooth during cleaning with the liquid jet. The example power spectra 1404, 1414 depict relative amplitude (in decibels) of the acoustic energy versus frequency. The example power spectrum 1404 schematically represents conditions early in the root canal cleaning process, when pulp and/or organic material substantially fill the canal spaces 30 of the tooth under treatment. The example power spectrum 1414 schematically represents conditions after the canal spaces 30 have been substantially cleaned of organic material and are at least partially filled with liquid. The changes between the power spectrum 1404 and the power spectrum 1414 provide acoustic signatures that may be monitored by the apparatus 300 to determine when cleaning has completed. For example, as schematically depicted in FIGURE 14, the power spectrum 1414 has increased in amplitude by an amount shown by arrow 1434. In some cases, a high frequency tail of the power spectrum 1404 may decrease in frequency by an amount shown by arrow 1424. Other signatures may exist including, for example, lower frequency tails of the power spectrum may change in frequency, portions of the power spectrum may change in shape, features (e.g., resonant frequencies) may increase/decrease in amplitude, etc.

In some treatment methods, the acoustic signatures shown in FIGURE 14 occur at audible frequencies such as, for example, frequencies between about 500 Hz and 5 kHz. For example, in one case, the amplitude shift 1434 may be about 10 dB and the frequency shift 1424 may be from about 3 kHz to about 2 kHz. As discussed above, the controller 312 may analyze the detected acoustic energy, and upon detection of one or more acoustic signatures indicative of completion of root canal cleaning, communicate a control signal to shut off the high speed liquid jet 60. In certain preferred embodiments, the apparatus 300 automatically communicates the control signal without requiring input or assistance from the dental practitioner.

### Crack Detection

Diagnosis of cracks in teeth is commonly made by an overall clinical assessment by a dental practitioner, because direct identification of the cracks via radiographical imaging is often ineffective at identifying cracks. Moreover, hairline cracks may be difficult to diagnose visually (with either visible or ultraviolet light) or radiographically (with dental X-rays).

Embodiments of the systems disclosed herein advantageously can be used to determine structural integrity of a tooth. For example, certain embodiments provide information on the presence or severity of a crack in a tooth. In certain embodiments, one or more dimensions of the crack may be determined. For example, the apparatus 100, 300 schematically depicted in FIGURES 4 and 6A may be used to transmit an acoustic signal into a tooth. In some embodiments, the acoustic signal comprises a relatively broadband, white noise acoustic ping. Acoustic energy propagating from the tooth is detected and analyzed for information related to a crack signature. The processor 206 shown in FIGURE 5 or the controller 312 shown in FIGURE 6A may be used to perform the analysis in some embodiments. The crack signature may represent reflected acoustic echoes from a crack and/or resonant oscillations of fluid in the crack (e.g., a Helmholtz resonance). In some cases, the crack signature comprises higher frequency acoustic signatures due to acoustic excitation of material in the crack.

### Endodontic Apparatus for Use with Liquid Jet Systems

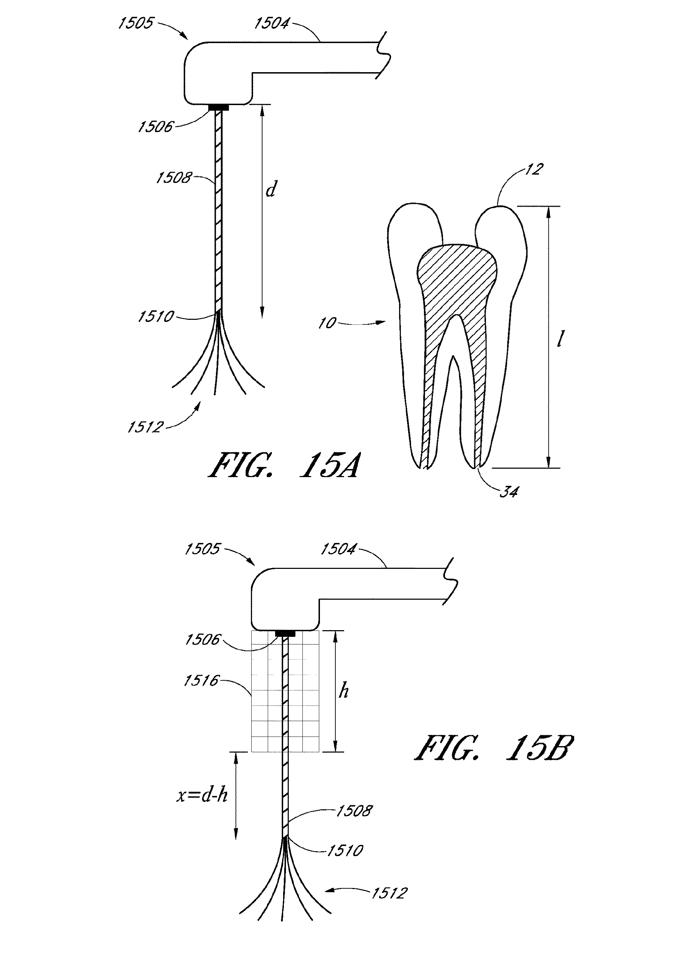

FIGURES 15A-15F schematically illustrate embodiments of apparatus that can be used with liquid jet systems. FIGURE 15A schematically depicts a handpiece 1504 that can be used by a dental practitioner to direct a collimated jet 1508 of liquid emitted from a nozzle 1506 at a distal end 1505 of the handpiece 1504. The collimated jet 1508 propagates a distance d from the nozzle 1506 before beginning to break up at a transition 1510 into a spray 1512 of liquid. In many embodiments of liquid jet apparatus, the transition 1510 between the collimated jet 1508 and the spray 1512 is relatively sharp (e.g., within a 1-2 cm). The distance *d* may be in a range from about a few centimeters to about 10 centimeters.

The collimated jet 1508 has a transverse width in a range from about 10 microns to about 1000 microns in various embodiments. In certain preferred embodiments, the transverse width is in a range from about 40 microns to 80 microns. The collimated jet 1508 has a speed in a range from about 100 m/s to about 300 m/s in various embodiments, and is about 220 m/s in a preferred embodiment. The collimated jet 1508 carries substantial kinetic energy and can readily cut tissue. After the transition 1510, the jet 1508 disperses into the spray 1512, which no longer retains the ability to cut tissue.

In preferred embodiments of the teeth cleaning methods described herein, the collimated jet 1508, rather than the spray 1512, is directed toward a dentinal surface of a tooth 10 in order to couple the kinetic energy of the jet 1508 into the tooth (e.g., to produce acoustic cavitation in the root canal spaces 30). Accordingly, the dental practitioner may position the handpiece 1504 so that a suitable dentinal surface is in the range of the collimated jet 1508. For example, the handpiece 1504 may be maneuvered until the nozzle 1506 is spaced from a dentinal surface by less than the distance d.

As depicted in FIGURE 15A, the tooth 10 can have a length *l*, measured, for example, from the apical foramen 34 to the occlusal surface of the crown 12. The length *l* may be measured from a dental X-ray taken of the tooth 10 with a suitable calibration member positioned in or adjacent the tooth 10. The length *l* need not be the full length of the tooth 10 (as depicted in FIG. 15A) and may be another suitable length (e.g., the size of the pulp chamber, the length of a root canal, etc.). In certain endodontic procedures, it may be advantageous to limit the range over which the collimated jet 1508 can impact portions of the tooth 10 such as, for example, the periapical regions near the foramen 34. Therefore, as schematically illustrated in FIGURE 15B, a spacer 1516 may be attached to the distal end 1505 of the handpiece 1504 to limit the range of the jet 1508. In the embodiment depicted in FIGURE 15B, the spacer 1516 comprises a cylindrical cage having a length h and formed from, for example, metal and/or plastic wires. The length x of the collimated jet 1508 that extends beyond the spacer 1516 is x = *d - h.* The use of the spacer 1516 prevents the distal end 1505 of the handpiece 1504 from being positioned too closely to the tooth 10 and effectively limits the "working range" of the collimated jet 1508 to be approximately the length x. In procedures in which it is desirable for the range x of the jet 1508 to be less than the length *l* of the tooth 10, the length h of the spacer 1516 may be selected to be greater than *d-l.* In certain embodiments, a kit that includes spacers 1516 having a variety of lengths h is provided so that the dental practitioner can select a suitable spacer 1516 for the particular jet length d and tooth size *l*.

An advantage of the spacer 1516 depicted in FIGURE 15B is that the wire cage only minimally obscures the vision of the dental practitioner. In another embodiment, the spacer 1516 is configured as a transparent or translucent annulus (formed from an elastomeric material for example). In other embodiments, the spacer 1516 comprises one or more elongated rods that extend away from the distal end 1505 of the handpiece 1504.

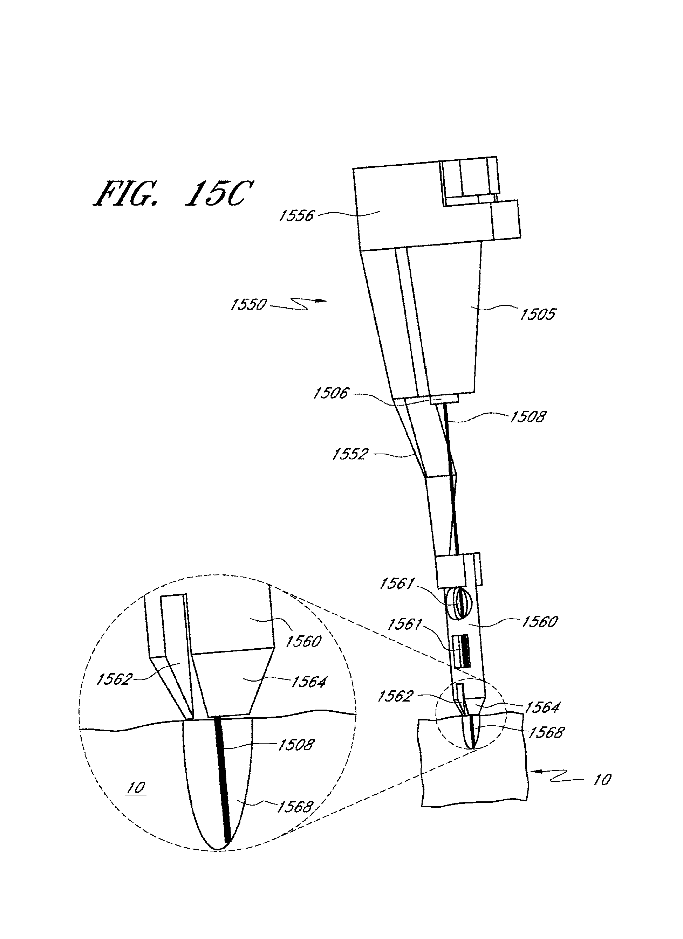

FIGURES 15C-15E schematically illustrate embodiments of an aiming element 1550 that may be attached at the distal end 1505 of a dental handpiece 1504. FIGURES 15C-15E schematically illustrate the distal end 1505 of the handpiece 1504 and do not show other portions of the handpiece 1504. FIGURES 15C-15E also include closeup views schematically illustrating embodiments of a distal end 1564 of the aiming element 1550 near a desired location 1568 in a tooth 10. The aiming element 1550 advantageously distances the jet 1508 from a tooth surface and also aids in aiming the jet 1508 toward the desired location 1568 in the tooth 10. The aiming element 1550 comprises an attachment portion 1556 and an aiming portion 1552. The attachment portion 1556 may be configured to mate with the distal end 1505 of the handpiece 1504. In some embodiments, the attachment portion 1556 clamps to the distal end 1505. The attachment portion 1556 advantageously may be configured so that the aiming element 1550 is removable from the handpiece 1504 so that differently sized and/or shaped aiming elements 1550 may be attached as desired by a dental practitioner.

The aiming portion 1552 of the aiming element 1550 may be elongated with a distal end portion 1564. The aiming portion 1552 may be offset from the jet 1508 to permit propagation of the jet 1508 from the nozzle 1506 to the desired location 1568 in the tooth 10. In the examples shown in FIGURES 15C-15E, the location 1568 is schematically depicted as a Gates-Glidden size-4 preparation. The aiming portion 1552 may be sized such that when the aiming element 1550 is attached to the handpiece 1504, the distance between the distal end 1564 and the nozzle 1506 is sufficiently short that the jet 1508 remains collimated until impact at the location 1568. The distance between the distal end 1564 and the nozzle 1506 may also be selected to be sufficiently large to provide the dental practitioner with good visibility while performing a dental procedure. In some embodiments, the aiming portion 1552 is configured so that the distance is in a range from about 5 mm to about 50 mm such as, for example, 20 mm.

FIGURE 15C schematically illustrates an embodiment of the aiming element 1550 comprising a tube potion 1560 that has a lumen that permits propagation of the jet 1508 to the location 1568 in the tooth 10. In some embodiments, the tube portion 1560 substantially surrounds the jet 1508. In other embodiments, the tube portion 1560 only partially surrounds the jet 1508 and may have a circumferential extent of, for example, about 270 degrees, about 180 degrees, or some other angular range. In the embodiment depicted in FIGURE 15C, the tube portion 1560 is ventilated and comprises one or more openings 1561 and/or notches 1562. The tube portion 1560 may be formed integrally with the aiming element 1550. In some embodiments, the tube portion 1560 is configured to slidably engage the aiming portion 1552, which advantageously permits the dental practitioner to select a suitably sized and/or shaped tube portion 1560 for the dental procedure.

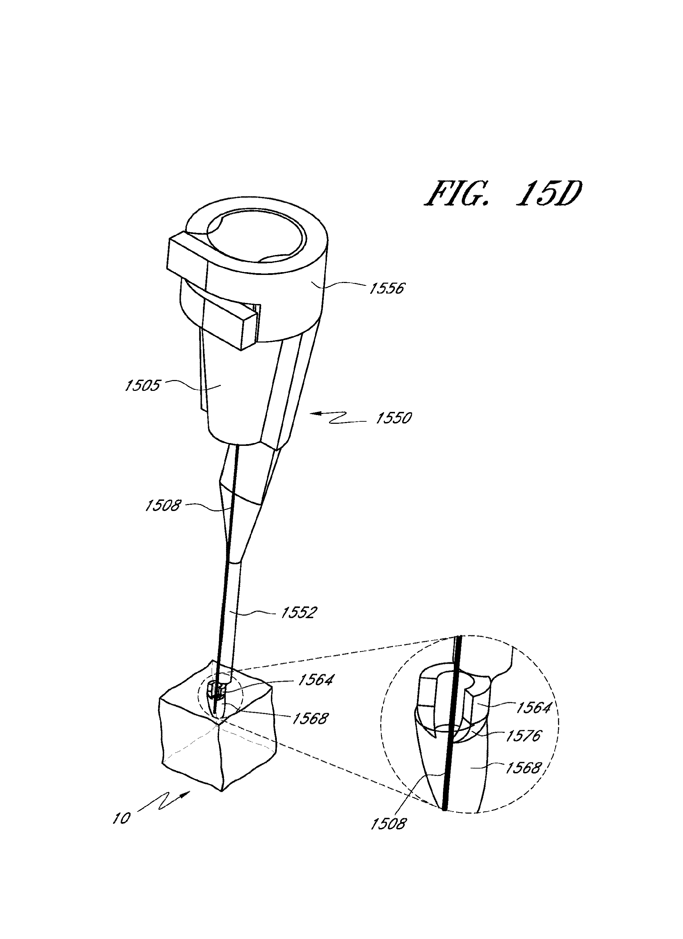

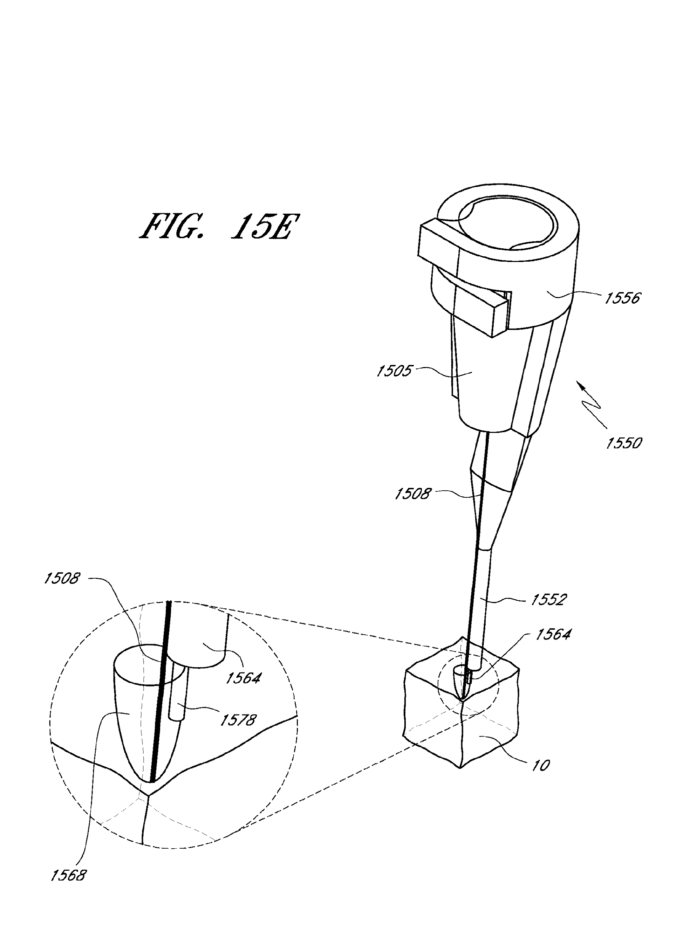

The distal end 1564 of the aiming element 1550 may be sized and/or shaped to engage the location 1568 of the tooth 10. For example, the distal end 1564 may have a size and/or shape to fit in a coronal opening of the tooth 10 (see FIG. 3). In the examples shown in FIGURES 15C-15E, the distal end 1564 is sized approximately as the diameter of an opening formed with a Gates-Glidden size-4 drill. Other sizes are possible. As shown in FIGURE 15C, the distal end 1564 may have a tip 1572 that is generally frustoconical. FIGURES 15D and 15E schematically depict other embodiments of the aiming element 1550. As shown in FIGURE 15D, the distal end 1564 may have a rounded tip 1576 that permits the aiming element 1550 to be swiveled around the location 1568. As shown in FIGURE 15E, the distal end 1564 may have an elongated tip 1578 (e.g., a pin) that can act as a pivot point to accurately aim the jet 1508 toward a desired target at the location 1568. If necessary during a dental procedure, the dental practitioner may apply pressure to urge the distal end 1564 (and/or the tip 1576, 1578) into or toward a target at the location 1568. In other embodiments, the distal end 1564 may have different tips than shown in FIGURES 15C-15E.

The aiming element 1550 advantageously may be fabricated from one or more durable, biocompatible materials such as, for example, polymers, stainless steel, and titanium. In some embodiments, the attachment potion 1556 and the aiming portion 1552 are fabricated from different materials.

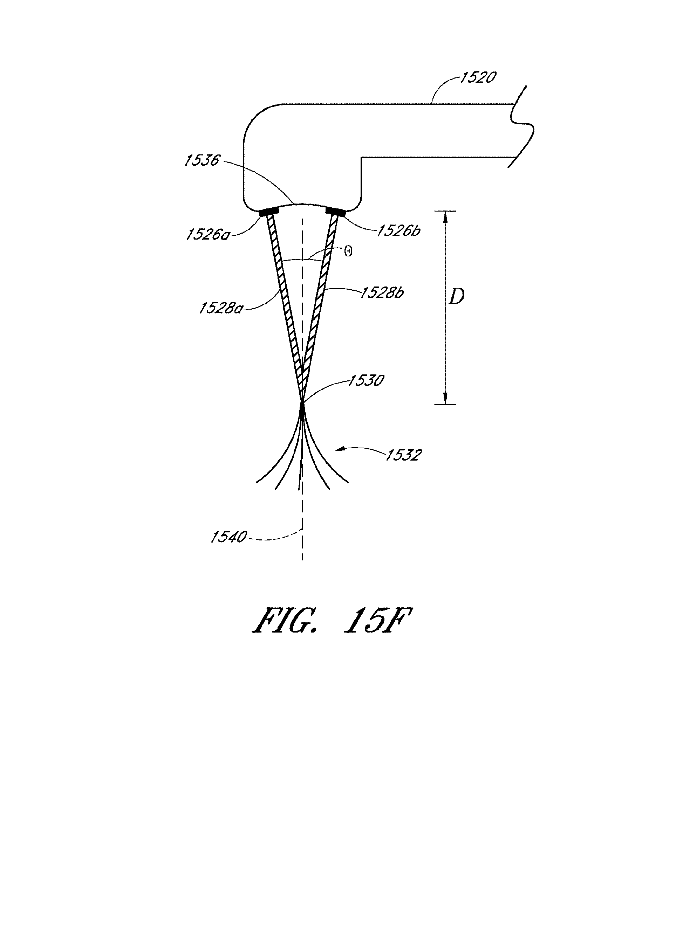

FIGURE 15F shows an embodiment of a handpiece 1520 configured to emit multiple beams 1528a, 1528b of liquid. The beams 1528a, 1528b emerge from nozzles 1526a, 1526b, respectively, that are disposed on a distal surface 1536 of the handpiece 1520. The distal surface 1536 can be shaped, for example by angling or contouring, so that the beams 1528a, 1528b are angled with respect to an axis 1540. The beams 1528a, 1528b propagate from the nozzles 1526a, 1526b, respectively, and intersect at a region 1530 beyond which the beams 1528a, 1528b break up into a spray 1532 of liquid. In this embodiment, the effective working range of the beams 1528a, 1528b is approximately the distance D shown in FIGURE 15F. The distance *D* may be in a range from about 5 mm to about 50 mm such as, for example, about 20 mm. In some embodiments, the orientation and/or position of the nozzles 1526a, 1526b on the distal surface 1536 is adjustable so that the working range D is adjustable. The orientation and/or position of the nozzles 1526a, 1526b can be selected so that the beams 1528a, 1528b form a suitable angle θ with respect to the axis 1540. For example, the beams 1528a, 1528b may be nearly parallel to the axis 1540 (e.g., θ is only a few degrees) so that the beams 1528a, 1528b can be directed into narrow openings in a tooth. The angle θ may be in a range from about 0.1 degrees to about 1 degree, from about 1 degree to about 5 degrees, from about 5 degrees to 30 degrees, or greater than 30 degrees in various embodiments.

In the embodiment illustrated in FIGURE 15F, the beams 1528a and 1528b are approximately symmetrically oriented with respect to the axis 1540 so that each beam 1528a, 1528b forms an angle of about θ/2 with the axis 1540. In other embodiments, the beams 1528a and 1528b are not symmetrically oriented about the axis 1540. For example, in some embodiments, one of the beams is directed substantially along the axis 1540 and the other beam is angled with respect to the axis 1540. Also, although two liquid beams 1528a and 1528b are illustrated in FIGURE 15F, in other embodiments, three, four, five, six, or more beams may be used.

In certain embodiments, each of the beams 1528a, 1528b is a collimated, high-speed liquid jet capable of cutting tissue. The beams 1528a, 1528b may have similar flow properties such as, for example, speed, diameter, and kinetic energy, or one or more of such flow properties may be different. In some embodiments, one of the beams is a lower speed liquid beam that does not have sufficient speed and/or energy to cut tissue. In such embodiments, the lower speed beam may have a larger diameter to permit easier alignment so that the beams 1528a, 1528b meet at the intersection 1530 and provide a desired range D.

### Methods of Operation of Liquid Jet Apparatus

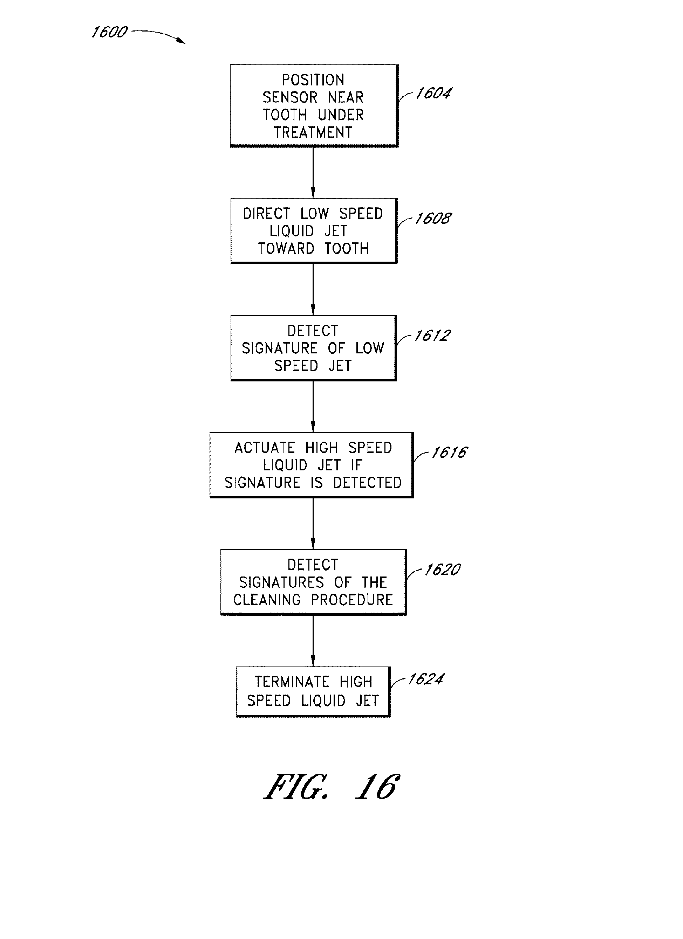

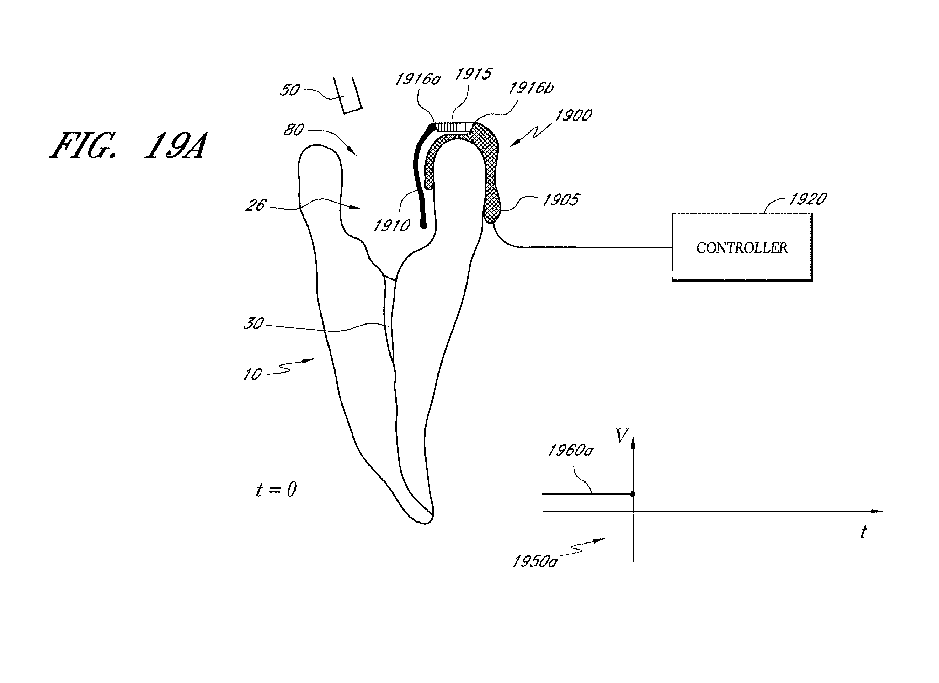

FIGURE 16 is a flow diagram that illustrates an example method of operation 1600 of a liquid jet apparatus that is used for an endodontic procedure as, for example, a root canal cleaning procedure. The method 1600 may be used, for example, with the liquid jet system 200 described with reference to FIGURE 5 in conjunction with the acoustic apparatus 100 described with reference to FIGURE 4 and/or the acoustic energy sensing apparatus 300 described with reference to FIGURE 6A. In other embodiments, the method 1600 may be used with a strain gage 1900 described below with respect to FIGURES 19A-19E. Portions of the method 1600 that require machine control may be implemented as executable instructions on a computer-readable medium such as, for example, volatile and/or nonvolatile memory, a magnetic drive, an optical drive, and so forth. The instructions may be executed by one or more special and/or general purpose computers so as to carry out the method 1600. In some embodiments, the processor 206 (FIG. 5) and/or the controller 312 (FIG. 6A) execute the instructions.

In block 1604, one or more sensors are positioned near the tooth that is to undergo treatment. For example, the sensors may include the acoustic elements 104 and/or the acoustic transducer 304. In other embodiments, the strain gage 1900 is used. In block 1608, the liquid jet apparatus is positioned so that a low-speed beam of liquid is directed toward the tooth under treatment. For example, the dental practitioner may position a handpiece (e.g., any of the handpieces shown in FIGS. 15A-15F) so that the low-speed flow of liquid impacts the tooth. It is preferable if the low-speed beam does not have sufficient speed or energy to cut tissue so that the dental practitioner may readily maneuver the beam in the mouth of the patient without substantial danger of harming tissue. For example, in some embodiments, the liquid jet apparatus produces a collimated liquid jet by flowing pressurized liquid through a small orifice. To produces a low-speed beam, the liquid jet apparatus may be operated at a pressure that is substantially lower than the pressure needed to produce a high-speed jet capable of cutting tissue. For example, in one embodiment, the high-speed beam is produced with a pressure of about 8000 psi, and the low-speed beam is produced with a pressure of about 3000 psi. Other pressures may be used in other embodiments.

In block 1612, the sensor (or sensors) may be used to detect a signature indicating that the low-speed liquid beam is impacting the desired tooth. For example, in some embodiments using acoustic sensors, if the magnitude of the acoustic energy propagating from the tooth is above a threshold, then the low-speed beam is assumed to be impacting the desired tooth. In some embodiments, an acoustic signature comprises detection of acoustic energy having frequencies in a predetermined frequency range or having a predetermined power spectrum. In embodiments using a strain gage, the signature may comprise a suitable voltage signal from the strain gage (see, e.g., FIGS. 19A-19E). If the signature is not detected, then audible, visible, and/or tactile commands may be provided to alert the dental practitioner that the beam is not impacting the desired tooth.

In block 1616, if an appropriate signature for the low-speed beam is detected, then there is a high probability that the low-speed beam is directed at the desired tooth, and the high-speed, collimated jet is actuated. For example, in pressurized liquid jet apparatus, the working pressure may be increased to the operational value (e.g., about 8000 psi in some embodiments). An advantage of the method 1600 is that the high-speed jet, which is capable of cutting tissue, is not actuated until the liquid beam is pointing to the desired tooth, which reduces the likelihood of harming tissue in the mouth or performing the procedure on the wrong tooth.

The length of time the high-speed, collimated jet is actuated may depend on the type of the procedure. For example, in procedures in which the high-speed jet couples energy into the tooth to cause acoustic cavitation, delamination of organic matter may occur in about 1 second to about 5 seconds, and the root canal spaces 30 may be rinsed and cleaned in about 5 seconds to about 10 seconds. Some embodiments of the liquid jet apparatus may shut off the high-speed jet after a predetermined time period. An advantage of jet systems that utilize embodiments of the method 1600 is that the system may monitor the treatment procedure and shut off the high-speed jet when the treatment is substantially complete and/or when a potentially dangerous condition is about to occur.

In block 1620, a sensor (or sensors) is used to detect a signature of the cleaning procedure. For example, in some embodiments the signature comprises and acoustic signature of acoustic energy from the tooth, and the system analyzes the detected acoustic energy for one or more acoustic signatures related to the endodontic procedure. The system may utilize the "passive listening" mode and/or the "pulse-echo" mode to determine one or more suitable acoustic signatures. Any one or more of the acoustic signatures described herein may be used by the system. For example, in the case of root canal cleaning procedures, the system may perform a correlation analysis of pulse-echoes as described with reference to FIGURES 9A-10 to determine the progress of the treatment. As depicted in FIGURE 10, when a pulse-echo correlation first decreases and then increases above a threshold, the treatment is substantially complete, and a shut-off signal may be communicated to the liquid jet apparatus to terminate the high speed liquid jet (block 1624). Another possible acoustic signature is described with reference to FIGURE 13. In this example, an event rate indicative of the rate of cavitation bubble formation and collapse may be used to determine the progress of the cleaning treatment. When the event rate decreases below a threshold, the cleaning is substantially complete and the high-speed liquid jet can be terminated (block 1624). Other acoustic signatures may be used such as changes in the power-spectrum of the detected acoustic energy described with reference to FIGURE 14.

In block 1620, detection of certain signatures may represent onset of a potentially unsafe and/or undesired condition. If such a signature is detected, the high-speed jet is terminated (block 1624), which advantageously reduces the likelihood of complications from the procedure. For example, in order to reduce the risk of infection to periapical tissue, it is beneficial if matter in the canal spaces 30 is not forced through the foramen 34. This matter may include organic matter to be cleaned from the canal spaces 30 as well as liquid from the jet. If matter begins to move through the canal spaces 30 (and potentially out through the foramen 34), the pattern of fluid flow may become substantially laminar, and the effects of acoustic cavitation (e.g., bubbles, jets, turbulence, etc.) may decrease. In some embodiments, the signatures may include one or more acoustic signatures. For example, an acoustic signature of laminar, non-turbulent flow may include an increase in the correlation of acoustic echoes reflecting from the canal spaces 30. For example, the pulse-echo trace may change from the time-variable, relatively low correlation trace shown in FIGURE 9A to the more steady, relatively high correlation trace shown in FIGURE 9B. In some embodiments, if an acoustic signature indicative of laminar flow is detected, then the jet is terminated to reduce the likelihood that the laminar flow will develop and force matter through the foramen. Other acoustic signatures of laminar flow may be used as well. For example, the acoustic energy propagated from the canal spaces 30 can have a different power spectrum when the canal spaces 30 are undergoing cavitation than when fluid is smoothly flow through the canal spaces 30.

### Bimodal Acoustic Receiver

FIGURE 17 schematically illustrates an embodiment of a bimodal acoustic receiver 1700 that is capable of detecting acoustic energy in both a low-frequency range and a high-frequency range. In some embodiments, the low frequency range includes audible frequencies below about 20 kHz, and the high frequency range includes ultrasonic frequencies above about 20 kHz. The high frequency range is from about 200 kHz to about 25 MHz in some embodiments of the acoustic receiver 1700.

The acoustic receiver 1700 comprises a high-frequency acoustic sensor 1704 and a low-frequency acoustic sensor 1706. The high-frequency acoustic sensor 1704 may comprise any suitable piezoelectric material such as, for example, a piezoelectric ceramic including, e.g., PZT (lead zirconate titanate), PLZT (lead lanthanum zirconate titanate), etc. The high-frequency sensor 1704 is configured to receive acoustic energy having frequency components in the high-frequency range. In embodiments using piezoelectric materials, the incoming acoustic energy causes small deformations of the receiver 1704, which are converted to electric signals through the piezoelectric effect. In various embodiments, the frequency response of the high-frequency sensor 1704 may be similar to the frequency responses shown in FIGURE 7A and/or FIGURE 11A. In such embodiments, the high-frequency acoustic sensor 1704 may not transmit acoustic energy having frequencies substantially below about 1 MHz. Although the embodiments of the high-frequency acoustic sensor 1704 has been described as a receiver, in other embodiments, the high-frequency sensor 1704 may also be operated to transmit acoustic energy so that the high-frequency acoustic sensor 1704 functions as an acoustic transceiver.

The low-frequency acoustic sensor 1706 may be attached to the high-frequency sensor 1704, for example, by bonding with a low-acoustic-attenuation adhesive material 1712. In the illustrated embodiment, the low-frequency sensor 1706 comprises an elongated member 1708 (for example, a metal wire) having a distal end 1716 that can be acoustically coupled to a tooth. Incoming acoustic energy having frequency components in the low-frequency range is transmitted as longitudinal and/or transverse vibrations of the elongated member 1708. In the illustrated embodiment, higher frequency acoustic energy is not transmitted by the elongated member 1708 due to, for example, poor impedance match between high-frequency energy and the elongated member and significant damping of high-frequency vibrations of the elongated member. In certain embodiments, the frequency response of the low-frequency sensor 1706 may be similar to the frequency response illustrated in FIGURE 12A.

As discussed above, acoustic signatures may be used to determine conditions within a tooth. The acoustic signatures may have sound frequencies in the high-frequency range and/or the low-frequency range. For example, certain pulse-echo mode acoustic signatures comprise sounds in the high-frequency range (see, e.g., FIGS. 8A-10), and certain passive listening acoustic signatures comprise sounds in the low-frequency range (see, e.g., FIG. 14). An advantage of apparatus and methods that utilize embodiments of the bimodal acoustic receiver 1700 is that the receiver 1700 has the capability of detecting both high-frequency and low-frequency acoustic signatures (if present).

### Materials for Coupling an Acoustic Transducer to a Tooth

In the apparatus and methods described herein, one or more acoustic elements are positioned near a tooth. The acoustic element may be an acoustic transducer that couples acoustic energy into the tooth (e.g., an ultrasonic pulse) and/or senses acoustic energy propagating from the tooth (e.g., reflected echoes of the pulse). Examples of acoustic elements have been described above with reference to FIGURES 6A and 6B. FIGURE 18A is a close-up view that schematically illustrates another embodiment of an acoustic element 1800a positioned near a surface 1802 of the tooth 10. This embodiment of the acoustic element 1800a comprises an acoustic transducer 1804 and an acoustic coupling tip 1808. The acoustic transducer 1804 may comprise one or more single- and/or multiple-element transducers such as, for example, piezoelectric transducers. The transducer 1804 may be operable to transmit and/or to receive acoustic energy. In certain embodiments, the acoustic transducer 1804 is configured to produce an acoustic pulse that is transmitted through the acoustic coupling tip 1808 and into the tooth 10. The acoustic coupling tip 1808 may be used as a relatively low-loss, impedance matching element between the acoustic transducer 304 and the tooth 10. In some embodiments, the tip 1808 is fabricated from a polymer material such as polycarbonate. The acoustic coupling tip 1808 may be configured as a signal delay line that provides a suitable time-delay between the transmitted pulse and reflected echoes from interfaces and structures in the tooth 10. The duration of the time-delay advantageously may be selected to reduce interference between the transmitted and reflected pulses. The shape of the tip 1808 may be selected to act as a waveguide that focuses and/or collimates the acoustic energy transmitted from the transducer 1808 so that a relatively high intensity acoustic pulse can be transmitted into the tooth 10. The shape of the tip 1808 also acts to guide acoustic energy propagating from the tooth 10 toward the transducer 1804 for detection (e.g., conversion to an electrical signal via the piezoelectric effect).

As schematically illustrated in FIGURE 18A, an acoustic coupling material 1812 may be interposed between the distal end of the acoustic coupling tip 1808 and the surface 1802 of the tooth 10, for example, to reduce undesired acoustic reflections between the tip 1808 and the tooth 10. In certain embodiments, an ultrasonic coupling gel is used. Many commercially available coupling gels have an acoustic impedance that is substantially different from the acoustic impedance of the materials in the acoustic coupling tip 1808 (e.g., polycarbonate) and the tooth (e.g., enamel, cementum, dentin). A possible disadvantage of such gels is that there may be substantial acoustic energy loss due to unwanted acoustic reflections at the interfaces where there is a substantial acoustic impedance mismatch (e.g., at the interface between the transducer tip 1808 and the gel and at the interface between the gel and the tooth surface 1802).

Accordingly, in certain embodiments, the acoustic coupling material 1812 is selected to have an acoustic impedance that reduces unwanted acoustic reflections so as to increase (or maximize) transmission of acoustic energy between the tip 1808 and the tooth surface 1802. As is known, a coupling material that has an acoustic impedance equal to the geometric mean of the acoustic impedances of the transducer tip 1808 and the tooth 10 may provide optimal acoustic transmission. Additionally, it may be advantageous for the coupling material 1812 to be substantially conformable (at least when the acoustic element 1800a is being maneuvered into position adjacent the tooth surface 1802) and to have substantially low acoustic attenuation (at least when the transducer 1804 is transmitting energy to and/or receiving energy from the tooth 10).

A coupling material 1812 that advantageously may be used with the apparatus and methods described herein comprises a flowable composite material. In certain embodiments, the flowable composite is a restorative that may be applied to a region of a tooth or restoration. The flowable composite may be light cured using a strong light source (such as an ultraviolet light). The flowable composite may be a hardenable adhesive comprising a filler material such as, for example, beads of silicon. The viscosity of the flowable composite in the pre-hardened state depends in part on the amount of filler material. The acoustic properties of the hardened material may depend in part on the amount of filler material. It is advantages in some embodiments for the hardness of the flowable composite (in the hardened state) to be close to the hardness of dentin and/or the hardness of the piezoelectric transducer material in order to provide efficient transmission of acoustic energy.

This coupling material is a flowable composite that may be applied to the tip 1808 before the acoustic element 1800a is inserted into the patient's mouth. Because the composite is a relatively viscous liquid gel when applied, the acoustic element 1800a may be suitably maneuvered until a desired position and orientation relative to the tooth surface 1802 are achieved. When in place, the composite can be hardened by application of ultraviolet light for a curing time of about 30 seconds in some embodiments. The hardened composite has an acoustic impedance that substantially matches the acoustic impedance of the tooth 10 so that interfacial reflection losses are reduced. The hardened material also acts as a waveguide for acoustic energy propagating between the tooth 10 and the acoustic element 1800a. Consequently, the hardened composite guides substantial amounts of acoustic energy between the tooth 10 and the transducer 1804 without excessive acoustic reflection and/or refraction losses, even in cases where the acoustic element 1800a is not oriented substantially orthogonally to the tooth surface 1802.

Use of the coupling material provides other advantages. For example, in certain embodiments, the acoustic attenuation coefficient for ultrasonic frequencies is much higher when the coupling material is in the flowable liquid gel phase than when the coupling material has hardened (e.g., by light-curing). Acoustic pulses transmitted from the transducer 1804 are substantially absorbed by the coupling material when in the gel phase and do not propagate into the tooth 10. Therefore, reflected echoes from the tooth will be nonexistent or will have very low amplitudes when the coupling material is in the gel phase. However, when the coupling material hardens, the acoustic attenuation drops significantly, and acoustic pulse energy will be transmitted to the tooth 10. The amplitudes of the reflected echoes will substantially increase in magnitude. Accordingly, in some methods for positioning the acoustic element 1800a, the transducer 1804 is used to transmit acoustic pulses while a dental practitioner is positioning the element 1800a near the tooth 10. An acoustic detection system (such as the apparatus 300 shown in FIG. 6A) is used to monitor the magnitude of reflected echoes from the tooth 10. When the acoustic element 1800a is in a suitable position and orientation, the dental practitioner begins to light-cure the material 1812. As the material 1812 hardens, the amount of reflected energy in the echoes will increase. By monitoring the increase in reflected echo energy, the dental practitioner will be able to monitor the progress of the hardening process. In certain embodiments, the acoustic detection system automatically monitors the energy in reflected echoes and provides a suitable signal (audible, visible, and/or tactile) to notify the dental practitioner that the hardening process is complete. For example, in certain embodiments the controller 312 of FIGURE 6A compares the magnitude of the reflected energy to a threshold in order to determine if the hardening process has completed.

FIGURE 18B schematically illustrates another embodiment of an acoustic element 1800b comprising an acoustic transducer 1804 and a housing 1816 configured to contain the acoustic coupling material 1812. In certain embodiments, a distal surface 1820 of the acoustic transducer 1804 is shaped (e.g., concave) to focus and/or collimate acoustic energy emitted by the transducer 1804. The housing 1816 may be fabricated from metal and/or plastic materials and may be shaped so that the acoustic coupling material 1812 (when inserted into the housing 1816) acts as a waveguide for acoustic energy. The housing 1816 may be configured as a wire cage or mesh that can hold the coupling material 1812. In other embodiments, the housing 1816 may be configured differently such as, for example, a frustoconical shell made from an elastomeric material. The acoustic coupling material 1812 advantageously may be a flowable composite (such as, e.g., Filtek™ flowable restorative available from 3M Corporation, St. Paul, Minnesota) that can inserted into the housing 1816, for example, by injection, and then suitably hardened. In certain embodiments, a kit having housings 1816 having a range of sizes and shapes is provided so that a dental practitioner can select a suitable housing 1816 for attachment to the transducer 1804. In other embodiments, the housing 1816 is not used and a portion of the acoustic coupling material 1812 is applied to the transducer 1804. In such embodiments, it is advantageous if the coupling material 1812 is suitably viscous.

In certain methods for using the acoustic element 1800b, the dental practitioner applies a sufficient amount of the acoustic coupling material 1812 to fill the housing 1816. The acoustic element 1800b is inserted into the patient's mouth and maneuvered as desired. When the acoustic element 1800b is in a suitable position and orientation, the coupling material 1812 is hardened, for example, by light curing. In certain embodiments, the acoustic element 1800b is a single-use component that is discarded after the dental procedure is completed. An advantage of the illustrated acoustic element 1800b is that the hardened coupling material 1812 forms in situ an acoustic coupling tip for guiding acoustic energy between the transducer 1804 and the tooth 10. In other embodiments, an acoustic coupling tip

(such as depicted in FIG. 18A) may also be used with the acoustic element 1800b, for example, to provide a structure that holds the transducer 1804 and to which the housing 1816 may be attached.

### Strain Gage Sensing Methods and Apparatus

FIGURES 19A-19E schematically illustrate a strain gage 1900 attached to an opening 80 in a tooth 10 during an example endodontic procedure with a liquid jet apparatus. The strain gage 1900 may be used to detect suitable signatures caused by flows of fluids near the tooth 10 (e.g., in the opening 80) during the procedure, and a controller 1920 advantageously may use such signatures for controlling the liquid jet apparatus (e.g., via the method 1600 described with reference to FIG. 16). Fluids that may be present during endodontic procedures include, for example, liquid delivered by the jet, irrigation liquid, liquefied organic matter, and so forth. In some procedures, fluids such as, for example, air (e.g., air entrained by the jet), compressed gases, and so forth may be present near the tooth 10, and in some embodiments, the strain gage 1900 may be used to detect suitable flow signatures of such fluids.

In the embodiment shown in FIGURES 19A-19E, the strain gage 1900 comprises a paddle 1910 coupled to a strain-sensing element 1915. The strain gage 1900 may be attached to the tooth 10 with a tooth clip 1905 (further described below). The paddle 1910 may be an elongated member formed from a substantially rigid material (e.g., a polymer and/or a biocompatible metal). In this embodiment, a proximal end of the paddle 1910 is coupled to a first end 1916a of the strain-sensing element 1915, and a distal end of the paddle 1910 extends at least partially into the opening 80 of the tooth 10 under treatment. A second end 1916b of the strain-sensing element 1915 is attached to the tooth clip 1905.

The strain-sensing element 1915 generates a signal in response to deformation (e.g., a change in length and/or curvature of the element). For example, the electrical resistance of the element 1915 may change as the element 1915 deforms under an applied stress. The change in resistance may be measured (e.g., using a Wheatstone bridge) and a corresponding voltage may be output to the controller 1920. Any suitable strain-sensing element 1915 (or combination of strain-sensing elements) may be used such as, for example, a metal foil strain sensor, a piezoelectric strain sensor, and so forth.

Forces applied to the paddle 1910 cause the paddle 1910 to deflect from its unstressed position shown in FIGURE 19A. Deflection of the paddle 1910 causes the strain-sensing element 1915 to deform (e.g., bend) and in response to generate a signal (e.g., a voltage) that is electrically communicated to the controller 1920. In the example endodontic procedure illustrated in FIGURES 19A-19E, liquid from the liquid jet apparatus is delivered to the opening 80, and flow of this liquid causes deflection of the paddle 1910. Flow of the liquid may include direct impact of the jet onto the paddle 1910 and/or swirling or turbulent fluid motions in the opening 80. As the paddle 1910 deflects under fluid stresses, the strain-sensing element 1915 responsively provides a signal indicative of the fluid flows in the opening 80 (as will be further described below). The strain gage 1900 may be electrically connected (using wired and/or wireless techniques) to the controller 1920, which processes the signals from the sensor 1900 in order to control the liquid jet. In some embodiments, signals from the sensor 1900 are output on a display (e.g., an oscilloscope) and may provide to the dental practitioner visual indications of fluid flows in the opening 80.

In certain embodiments, the signal from the strain gage 1900 may be processed by, for example, amplification, digitization, sampling, filtering, and/or other signal processing techniques. The processing may be performed by the controller 1920 and/or other electronic components. Signatures of the fluid flows in the opening 80 may be determined using signal processing techniques including, for example, signal correlation, Fourier transform, wavelet analysis, and so forth. In some embodiments, in addition to the strain gage 1900, one or more acoustic sensors are used to detect acoustic signatures (as described herein) caused by the jet.

FIGURES 19A-19E schematically illustrate use of the strain gage 1900 during an example endodontic procedure with a liquid jet apparatus. The liquid jet apparatus comprises a handpiece 50 positioned to deliver a liquid jet 1930, 1940 into the opening 80 in the tooth 10. In this example, the opening 80 has been formed in the tooth 10 to provide access to the pulp cavity 26 and/or the canal space 30. The opening 80 may be a coronal opening (as depicted in FIGS. 19A-19E). In other procedures, the opening 80 may be in the buccal or lingual surfaces of the tooth 10, for example. Multiple openings are used in some procedures.

As discussed above with reference to FIGURE 16, the high-velocity liquid jet 1940 may have a velocity that is sufficiently high to cut tissue in the patient's mouth. Therefore, in some procedures it is beneficial for the high-velocity jet 1940 to be actuated only after the dental practitioner has suitably directed a low-velocity jet 1930 (e.g., with insufficient velocity to cut tissue) into the opening 80 of the tooth under treatment. Accordingly, in these procedures, the controller 1920 actuates the high-velocity jet 1940 only after the strain gage 1900 detects the presence of the low-velocity liquid jet 1930 in the opening 80 of the tooth 10.

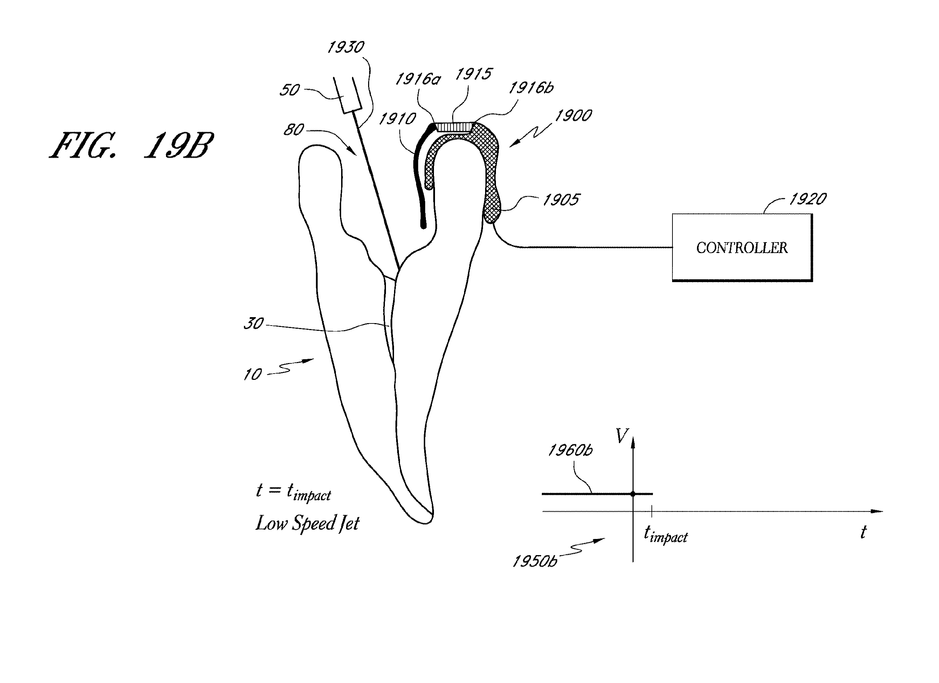

FIGURES 19A-19E schematically show a time sequence of an example procedure using the liquid jet apparatus to direct a low-velocity jet 1930 and a high-velocity jet 1940 into the opening 80 in the tooth 10. FIGURE 19A schematically illustrates the dental procedure before a liquid jet is actuated (at time t=0), and FIGURES 19B-19E schematically show the endodontic procedure at subsequent times.

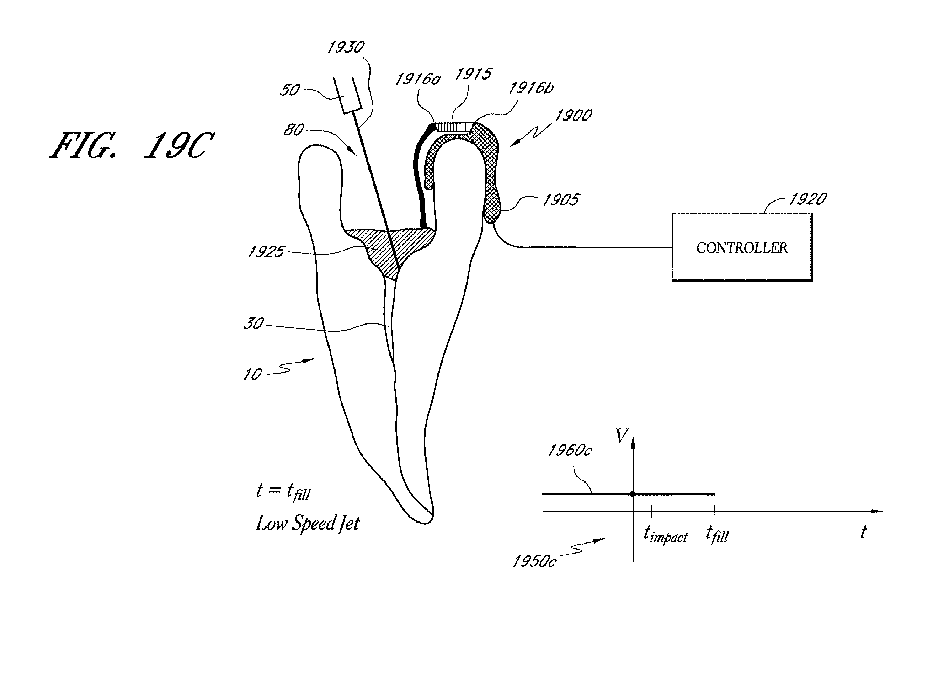

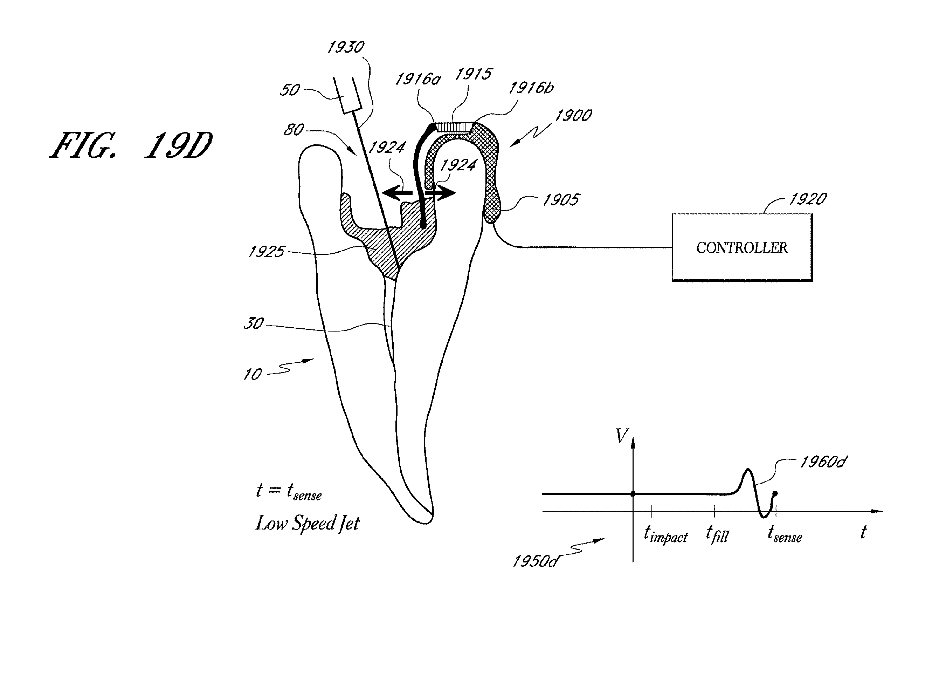

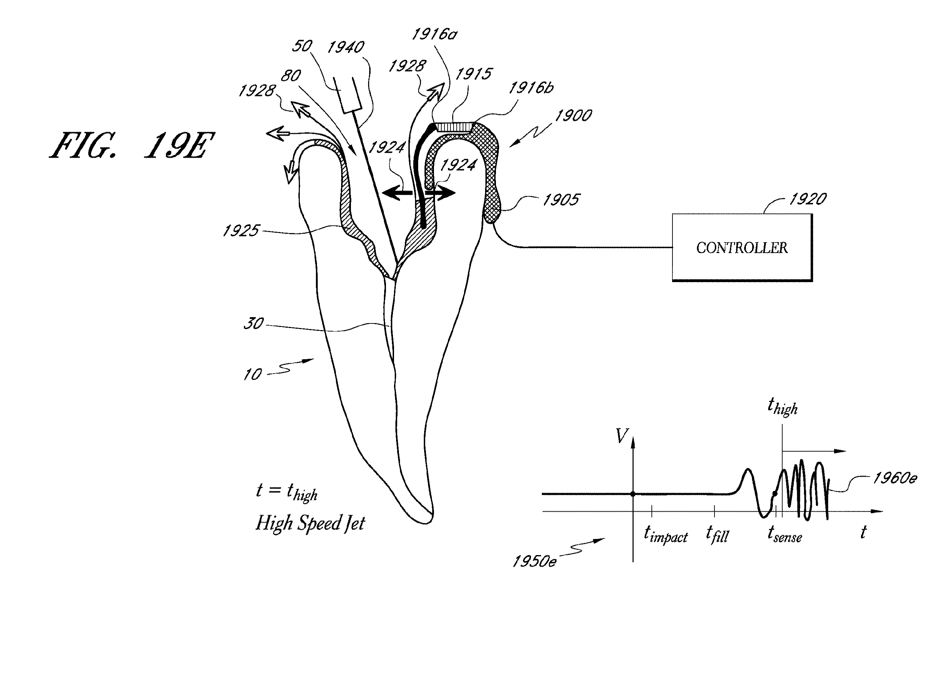

In the example procedure schematically shown in FIGURES 19A-19E, the low-velocity jet 1930 is actuated at time t=0 (FIG.19 A). The low-velocity jet 1930 impacts a dentinal surface in the opening 80 at time t_{impact} (FIG. 19B), and the opening 80 begins to fill with liquid 1925 (FIG. 19C). At time t_{fill}, the liquid 1925 reaches the distal end of the paddle 1910 (FIG. 19C). The opening 80 continues to fill with liquid 1925, and fluid flows in the opening 80 cause the paddle 1910 to deflect, which deforms the strain-sensing element 1915. The strain gage 1900 outputs to the controller 1920 a signal indicative of the deformation of the strain-sensing element 1915 (FIG. 19D). At the time tₛₑₙₛₑ, a sufficient flow of fluid in the opening 80 has been detected that there is a sufficiently high likelihood that the low-velocity jet 1930 has been properly delivered to the opening 80 in the tooth 10 under treatment. Accordingly, at time t_{high}, the controller 1920 actuates the high-velocity jet 1940 (FIG. 19E). Delivery of the high-velocity jet 1940 into the opening 80 may generate a more turbulent fluid flow in the opening 80 and may, in some cases, cause liquid and/or organic material to be ejected from the opening 80 (indicated by arrows 1928). As described above, the high-velocity liquid jet 1940 may provide root canal cleaning by inducing acoustic cavitation in the canal spaces 30.

FIGURES 19A-19E also include graphs 1950a-1950e, respectively, which plot example signal traces 1960a-1960e output by the strain gage 1900 as a function of time (t). In the example schematically illustrated in these figures, the signal traces 1960a-1960e represent a voltage (V) output by the strain gage 1900. In other embodiments, the signal traces 1960a-1960e may represent a current, a resistance, an impedance, a capacitance, or other signal output by the strain gage 1900.

In this embodiment, when the paddle 1910 is undeflected, the strain gage 1910 outputs a steady, non-fluctuating voltage signal, which is schematically shown as the "flat line" in the signal traces 1960a-1960c in FIGURES 19A-19C. As discussed above, fluid flows in the opening 80 cause the paddle 1910 to deflect after the time t_{fill}, and the signal trace 1960d indicates the deflection as a fluctuating voltage between the time t_{fill} and tₛₑₙₛₑ (FIG. 19D). At the time tₛₑₙₛₑ, a sufficient voltage signal has been detected by the processor 1920 to indicate that liquid from the low-velocity jet 1930 is in the opening 80, and at time t_{high} the high-velocity jet 1940 is actuated (FIG. 19E). After the high-velocity jet 1940 impacts dentinal surfaces and/or organic material in the opening 80, the signal trace 1960e may fluctuate more rapidly (and/or with higher amplitude) than during the time when the low-velocity jet 1930 was actuated (e.g., before the time t_{high}). Therefore, the strain gage 1900 advantageously may be used to provide signatures of the low-velocity jet 1930 and/or the high-velocity jet 1940. The controller 1920 may be configured to use the signatures for control of the liquid jet apparatus.

### Tooth Clips

As discussed above, the strain gage 1900 may be coupled to the tooth 10 using a tooth clip 1905. The tooth clip 1905 may be sufficiently small that the clip 1905 does not interfere with the dental practitioner's view of or access to the treatment site. The tooth clip 1905 may be positioned in a variety of orientations relative to the tooth 10. The orientations may be selected depending on, for example, the size, shape, and/or or location of the opening 80, the amount of space in the patient's mouth, the type of dental procedure, and/or the dental practitioner's preferences. For example, the tooth clip 1905 may be positioned on the buccal, the lingual, the mesial, the distal, or the palatal side of the tooth 10. In some procedures, more than one tooth clip 1905 is used to hold one or more sensors. In certain embodiments, a kit comprising tooth clips 1905 in a range of sizes and/or shapes is provided so that a dental practitioner can select a clip 1905 to accommodate variations in tooth anatomy and presentation.

In some embodiments, the clip 1905 may comprise a curved portion having, for example, a "U"-shaped cross-section as schematically shown in FIGURES 19A-19E. The clip 1905 may be formed from a resilient material (e.g., an elastomer or a biocompatible metal) such that the legs of the "U" provide a retaining force when clipped to the tooth 10. In some embodiments, the curved portion of the clip 1905 comprises one or more "U"-shaped wire elements. In certain embodiments, curved portion of the clip may have a "C"-shape, a tear drop shape, or some other suitable shape. For placement on the tooth 10, a dental practitioner may stretch open the legs of the clip 1905 and place the legs over the tooth (e.g., with one leg in the opening 80 and one leg on an outer tooth surface). The resilient forces of the material comprising the clip permit the legs to spring back against the surfaces of the tooth. In some embodiments, pads may be disposed at the ends of the legs to provide better grip and/or to reduce damage to the tooth surfaces.

Embodiments of the tooth clip 1905 may be configured to be secured to a tooth using a variety of techniques. Some embodiments of the clip 1905 comprise a small cam to lock on to the tooth preparation. The cam squeezes against the tooth and the clip to create a contact force securing the clip 1905 in place once it has been positioned as desired. In some embodiments, the tooth clip 1905 comprises a set screw, that when turned provides contact pressure against a surface of the tooth. The contact pressure urges the clip against an opposing tooth surface, thereby securing the clip 1905 to the tooth. The tooth clip 1905 may be removed by turning the screw in the opposite direction to release the contact pressure.

In certain embodiments, the tooth clip 1905 is formed using a material with a high yield strength (e.g., spring steel) so that the clip 1905 will return to its original shape after significant deformation (such as being bent to fit a tooth preparation). Superelastic material (e.g., nickel titanium, nitinol, etc.) may also be used. The "U"-shaped clip embodiments described above advantageously may be formed from such materials.

In some embodiments, the tooth clip 1905 is formed using a shape-memory alloy (e.g., nickel titanium). The shape-memory alloy has a low temperature martensitic phase in which the alloy is relatively soft. The martensitic phase occurs when the clip is cooled below a transition temperature, which may be below room temperature for some alloys. When the alloy is in the soft, martensitic phase, the tooth clip 10 may be bent, twisted, and/or shaped as desired by a dental practitioner to fit a patient's tooth. As the clip warms above the transition temperature (e.g., toward room temperature), the alloy experiences a transformation to a harder, austenitic phase. In the austenitic phase, the material returns to (e.g., "remembers") its original shape, which may be selected to provide a retaining force on the tooth. To remove the tooth clip 10, the alloy may be cooled below the transition temperature for transformation to the soft, martensitic phase.

In certain embodiments, the tooth clip 1905 is formed as a unitary structure. In other embodiments, the tooth clip 1905 comprises an inner element to be positioned inside the opening 80, and an outer element to be positioned on an outer surface of the tooth 10. The inner and outer elements may be secured to each other and to the tooth 10 by a worm screw coupled to upper ends of the inner and outer elements. By turning the worm screw, the upper ends are pushed apart, while lower ends of the elements are pushed against tooth surfaces. In other embodiments, the inner and outer elements may be configured similarly to an adjustable wood clamp, in which one (or more) screws are turned to bring the elements toward each other so as to clamp onto the tooth 10.

FIGURES 19A-19E schematically illustrate an embodiment of the tooth clip 1905 used to attach the strain gage 1900 to the tooth 10. In other embodiments, the tooth clip 1905 may be used to attach other devices, apparatus, and/or detectors to the tooth 10 such as, for example, an acoustic sensor.

Although the tooth 10 schematically depicted in the many of the figures is a molar, one of ordinary skill in the art will appreciate that the procedures may be performed on any type of tooth such as an incisor, a canine, a bicuspid, or a molar. Also, the disclosed methods are capable of detecting structures and movement in, as well as energy from, root canal spaces having a wide range of morphologies, including highly curved root canal spaces which can be difficult to image and/or view using conventional dental techniques. Moreover, the disclosed methods may be performed on human teeth (including juvenile teeth) and/or on animal teeth.

The foregoing description sets forth various preferred embodiments and other illustrative, but non-limiting, embodiments of the inventions disclosed herein. The description provides details regarding combinations, modes, and uses of the disclosed inventions. Other variations, combinations, modifications, equivalents, modes, uses, implementations, and/or applications of the disclosed features and aspects of the embodiments are also within the scope of this disclosure, including those that become apparent to those of skill in the art upon reading this specification. Additionally, certain objects and advantages of the inventions are described herein. It is to be understood that not necessarily all such objects or advantages may be achieved in any particular embodiment. Thus, for example, those skilled in the art will recognize that the inventions may be embodied or carried out in a manner that achieves or optimizes one advantage or group of advantages as taught herein without necessarily achieving other objects or advantages as may be taught or suggested herein. Also, in any method or process disclosed herein, the acts or operations making up the method or process may be performed in any suitable sequence and are not necessarily limited to any particular disclosed sequence.

## Claims

1. An apparatus for removing organic material from a root canal of a tooth, the apparatus comprising:
a liquid jet assembly configured to produce a high velocity beam of liquid capable of cleaning the root canal of organic material;
a sensor configured to detect completion of the cleaning and, in response, to produce a signal; and
a controller configured to communicate a shut-off signal to the liquid jet assembly upon receipt of the signal from the sensor based on an acoustic signature, the acoustic signature comprising: cavitation-induced effects, how an amplitude of the signal changes during the cleaning, a high-frequency acoustic signature, or a low-frequency acoustic signature.

2. The apparatus of Claim 1, wherein the sensor comprises an acoustic sensor.

3. The apparatus of Claim 1, wherein the controller is configured to automatically communicate the shut-off signal to shut off flow of the high-velocity beam.

4. The apparatus of Claim 1, wherein the apparatus is configured to provide a user with varying degrees of control over the shut-off signal.\

5. The apparatus of Claim 1, wherein the controller is configured to generate an image of the tooth to be output on a display.

6. The apparatus of Claim 1, further comprising an acoustic transducer.

7. The apparatus of Claim 1, wherein the sensor is configured to operate in a pulse-echo mode.

8. The apparatus of Claim 1, wherein the sensor is configured to operate in a passive listening mode.

9. The apparatus of Claim 1, wherein the sensor is configured to measure a thickness of dentin in the tooth.

10. The apparatus of Claim 1, wherein the acoustic signature comprises the high-frequency acoustic signature, the high-frequency acoustic signature comprising acoustic frequencies in a range from about 200 kHz to about 25 MHz.

11. The apparatus of Claim 1, wherein the acoustic signature comprises the low-frequency acoustic signature, the low-frequency acoustic signature comprising acoustic frequencies in a range from about 10 Hz to about 10 kHz.

12. The apparatus of Claim 1, wherein the acoustic signature comprises the low-frequency acoustic signature, the low-frequency acoustic signature comprising a Helmholtz resonant frequency of the tooth.

13. The apparatus of Claim 1, wherein the acoustic signature comprises how an amplitude of the signal changes during the cleaning.

14. The apparatus of Claim 13, wherein the acoustic signature comprises a first power spectrum representing conditions early in the cleaning and a second power spectrum representing conditions after canal spaces have been substantially cleaned of organic material.

15. The apparatus of Claim 15, wherein the acoustic signature comprises acoustic frequencies between about 500 Hz and 5 kHz.
